# FASCICULE DE BREVET EUROPEEN

(11) **EP 0 550 740 B1**
(45) Date de publication et mention de la délivrance du brevet: **25.09.1996**
(21) Numéro de dépôt: 92916944.9
(22) Date de dépôt: 22.07.1992
(51) Int. Cl.: G01N 33/543, C12M 3/00, G01N 33/50

(54) **DOSAGE IN VITRO DE MOLECULES AU NIVEAU DE LEUR SITE DE PRODUCTION PAR DES CELLULES EN CULTURE**
In vitro Analyse von Molekülen an ihrer Produktionsstelle durch die Zellkultur
IN VITRO ASSAY OF MOLECULES AT THEIR PRODUCTION SITE BY CULTURED CELLS

(30) Priorité: 23.07.1991 FR 9109303
(43) Date de publication de la demande: 14.07.1993
(73) Titulaire: BioSource Europe S.A., 6220 Fleurus (BE)
(72) Inventeur: DE GROOTE, Donat, B-1410 Waterloo (BE); BAUDRIHAYE, F., Marc, B-1410 Waterloo (BE); DELACROIX, Dominique Village Debat, F-92380 Garches (FR); FRANCHIMONT, Paul, B-5280 Modave (BE)
(74) Mandataire: Warcoin, Jacques
(86) Numéro de dépôt international: FR9200719
(87) Numéro de publication internationale: WO9302361

(56) Documents cités:
- EP-A- 0 146 654
- EP-A- 0 202 975
- EP-A- 0 350 233
- WO-A-91/09970
- US-A- 3 572 892
- WPIL/DERWENT, week 8744, Derwent Publications Ltd., London, GB; AN 87-310217

## Description

La présente invention concerne un procédé d'évaluation de la production par des cellules vivantes de molécules telles que des médiateurs solubles ou des éléments de particules infectieuses, dans lequel on effectue une culture cellulaire, et éventuellement une stimulation, pour induire ladite production, et l'on évalue la quantité de molécules produites par dosage immunologique sur phase solide in vitro.

La biologie clinique joue un rôle considérable dans l'évaluation médicale des états pathologiques, et ce à tous niveaux (diagnostic, pronostic, suivi thérapeutique, etc...). Un domaine privilégié de la biologie clinique est le dosage de médiateurs circulants, notamment des hormones, qui sont présents au niveau plasmatique, par exemple toutes les hormones peptidiques ou stéroïdiennes. Les procédures d'analyse préférentielles de ces paramètres reposent sur des méthodes de dosage immunologiques dont il existe un grand nombre de variantes bien connues de l'homme de l'art: dosage par compétition ou en sandwich, en phase liquide ou sur phase solide, en particulier des dosages, RIA, IRMA, ELISA, FIA-DELFIA.

Au cours des dernières années, de nombreux médiateurs biologiques solubles ont été mis en évidence, qui jouent un rôle déterminant dans l'équilibre de l'organisme ou les déséquilibres qui correspondent aux états pathologiques, mais qui ne sont pas normalement présents dans le plasma. Ces médiateurs sont de nature peptidique (neuropeptides, cytokines, etc...) ou non. Ces médiateurs n'étant pas des composants habituels du plasma en quantités mesurables et significatives, l'exploration des états normaux ou pathologiques par l'évalutation de ces médiateurs implique des approches nouvelles et des modes de réalisation particuliers, adaptés au travail routinier des laboratoires de biologie clinique notamment.

Afin de mieux illustrer ces notions, on prend l'exemple des cytokines en général, incluant lymphokines, monokines, interferons, facteurs de croissance, facteurs de différenciation, etc... . Les cytokines sont produites par différentes cellules de l'organisme et notamment le plus souvent, par les cellules du sang. La plupart des cytokines ont un mode d'action qui est essentiellement de type autocrine ou paracrine alors que leur activité endocrine est limitée. Dès lors, les taux de cytokines qui peuvent être mesurés dans le plasma ou le sérum des individus sains sont généralement en dessous des limites de détection des dosages biologiques ou immunologiques classiques. C'est seulement dans des situations pathologiques aiguës que des taux circulants de certaines cytokines peuvent être détectés dans le sérum ou le plasma, correspondant à une action endocrine de ces cytokines. On peut citer l'action endocrine de l'IL6 sur le foie pour induire la production des protéines de la phase aiguë ou sur le cerveau pour induire la fièvre lors d'une infection ou d'un traumatisme. De façon générale, les taux plasmatiques ou sériques des cytokines sont un bon reflet de leur activité endocrine mais un mauvais reflet de leurs activités autocrine et paracrine. Pourtant, cette activité paracrine est déterminante pour le bon fonctionnement de l'organisme. Des stimulations anormales (par exemple lors d'infections virales bactériennes ou parasitaires; lors de traumatismes ou de brûlures ou suite à l'ingestion de médicaments ou de toxiques; ou encore après contact avec un allergène, etc...) ou des dysfonctionnements pathologiques de l'organisme (maladies cancéreuses ou dégénératives; maladies inflammatoires ou auto-immunes; déficit génétiques, etc...) peuvent conduire à des anomalies de production des cytokines. On peut ainsi étudier les cellules productrices de cytokines et démontrer des excès ou des déficits de production. La capacité, par une cellule productrice, de répondre à une stimulation donnée par une production de cytokines permet d'évaluer l'état fonctionnel de la cellule (type et quantité de cytokines produites, cinétique de production, sensibilité à la stimulation, nécessité ou non de cofacteurs à la stimulation, etc...). On peut ainsi, par exemple, définir des états d'activation ou de préactivation des cellules qui peuvent avoir des significations diagnostiques ou pronostiques très importantes (par exemple, risque d'encourir un choc septique dans une situation donnée). La signification biomédicale de ces mesures de "capacités de production" est différente et complémentaire de la mesure des cytokines plasmatiques ou sériques. Les informations obtenues par ce type de test ne peuvent donc être obtenues par un simple dosage plasmatique ou sérique.

Cette voie d'exploration dont le potentiel est très important a été peu utilisée jusqu'à présent, et uniquement au niveau de laboratoires d'investigations et de recherche, en raison de la complexité technique de ces mesures.

En effet, jusqu'à présent, la mesure de la capacité de production des cellules mononuclées était réalisée lors d'une procédure multiétapes impliquant:
1. l'isolement et la purification des cellules visées;
2. la mise en culture de ces cellules dans un milieu artificiel;
3. le prélèvement d'un aliquot du milieu de culture pour un dosage conventionnel biologique ou immunologique;
4. la réalisation séparée du dosage dans un format technologique approprié tel un kit d'immunodosage. Le document WO 91/09970 illustre l'état de la technique.

Plusieurs problèmes sont liés à ces différentes étapes:
- l'isolement et la purification des cellules entraînent une sélection plus ou moins volontaire des différentes sous-populations de cellules sanguines. Les rendements d'une purification à une autre peuvent être différents. Les processus d'isolements et de purifications peuvent par eux-mêmes entraîner une activation non contrôlée des cellules.
- la mise en culture dans des milieux artificiels prive les cellules des interactions cellulaires et moléculaires qui sont présentes dans le sang complet et participent à la régulation des activations cellulaires.
- la procédure multiétapes est fastidieuse et peu automatisable car après culture des cellules, les surnageants doivent être récupérés et éventuellement centrifugés pour éliminer les éléments cellulaires après quoi ils sont dosés (soit directement, soit après stockage sous forme congelée) par les méthodes biologiques ou immunologiques (enzymo ou radio-immunologiques) disponibles.

D'une manière générale, ces difficultés se reproduisent chaque fois que l'on veut évaluer la production d'un médiateur soluble par des cellules, in vitro à l'aide d'un immunodosage.

L'invention a pour objet un procédé d'évaluation, par méthode immunologique, de la production de molécules telles que des médiateurs solubles ou des éléments de particules infectieuses, notamment virales, par des cellules vivantes in vitro, caractérisé en ce que la culture cellulaire au cours de laquelle s'effectue la production et la phase d'immunocapture du dosage immunologique sont réalisés au sein d'un seul et même complexe récipient-support.

En d'autres termes, le support de la culture et le support de la réaction de dosage immunologique sont tous deux présents ensemble au cours de la phase de production de la molécule à doser au lieu d'être dissociés dans des phases successives du processus normal d'évaluation.

L'invention concerne également un format technologique conçu et adapté pour ce procédé d'évaluation, notamment tout format comprenant un support à la culture cellulaire dans lequel est déjà intégré un élément du dosage, notamment sous forme d'anticorps fixés sur une phase solide du support ou une phase solide telle que des billes à l'intérieur du support. En dehors de ce support, le format peut comprendre tous milieux ou réactifs utiles à la culture et au dosage in situ sur le site de production en fonction de l'application poursuivie.

Le procédé de dosage in situ, et le format technologique correspondant, qui intègre la culture, avec ou sans la stimulation de la population cellulaire étudiée, et l'immunocaptation nécessaire au dosage en une seule étape au niveau du site de production consiste à cultiver la population cellulaire à étudier par exemple dans les puits d'une plaque de microtitration en présence des éléments nécessaires à la stimulation et des éléments nécessaires au dosage d'intérêt. Au moment choisi par l'utilisateur, la culture est interrompue par lavage de la phase solide et le dosage est directement révélé dans la plaque sans transferts du milieu de culture.

Plus précisément, la présente invention a donc pour objet un procédé d'évaluation de la production par des cellules vivantes de molécule(s) telle(s) que des médiateurs solubles, ou des éléments de particules infectieuses notamment virales, dans lequel on effectue une culture cellulaire, et éventuellement une stimulation, pour induire ladite production, et l'on évalue la quantité de molécule(s) produite(s) par dosage immunologique sur phase solide in vitro, caractérisé en ce que:
a) la culture cellulaire et éventuellement la stimulation sont effectuées, éventuellement en présence du traceur, dans un récipient qui incorpore la phase solide du dosage sur laquelle sont fixés les éléments de capture spécifiques de la (ou des) molécule(s) à doser, et en ce que
b) lorsque le temps de culture est écoulé, après lavage de la phase solide et incubations des éventuels différents autres réactifs nécessaires au dosage, notamment un substrat du traceur en présence de la phase solide, on lit le signal émis par le traceur fixé à la phase solide ou par son dit substrat.

De préférence, dans l'étape b), le traceur et la phase solide sont incubés dans le récipient ayant servi à la culture.

Lorsque le traceur est marqué par un enzyme, lesdits réactifs nécessaires au dosage comprennent en effet un substrat de l'enzyme que l'on incube après ledit lavage de la phase solide et on lit alors le signal émis plus précisément par le substrat.

Par "incorporer", on entend dans la présente demande que le support solide du dosage peut être physiquement dépendant du support de la culture (par exemple fond du puits ou du tube de culture) ou physiquement indépendant (par exemple billes ou sphères ajoutées sur le support de culture), l'important étant la présence simultanée des supports de la culture et du dosage.

Par récipient de culture, on entendra désigner le récipient dans lequel est effectuée la culture, il pourra ou non être la phase solide sur laquelle est effectuée l'immuno-captation.

On a en effet découvert de façon tout à fait surprenante que les éléments et réactifs intervenant dans la culture et les éléments et réactifs nécessaires au dosage:
- d'une part étaient stables et parfaitement compatibles lorsqu'ils sont mis en présence et mis en oeuvre dans le même récipient-support dans les conditions de culture, que ce soit notamment pour les éléments de captures fixes à la phase solide ou pour le traceur, et
- d'autre part, n'interféraient pas négativement sur la qualité de la culture et de la production des molécules à doser.

Dans certains cas, notamment lors d'un dosage de type sandwich, le traceur peut être mis à incuber en présence de la phase solide après un premier lavage de celle-ci intervenant après la production de la molécule à doser par culture cellulaire et son immuno-captation. Après incubation du traceur et un deuxième lavage de la phase solide, on effectue la lecture du signal émis après incubation éventuelle avec les autres réactifs nécessaires au dosage.

Selon une variante tout à fait avantageuse du procédé selon l'invention, la phase solide consiste dans la surface intérieure du récipient de culture sur laquelle sont fixés notamment par absorption ou par lien chimique les éléments de capture spécifiques de la ou des molécules à doser.

Selon une autre variante, la phase solide du dosage est constituée par un ou des éléments physiquement indépendants des récipients de culture cellulaire. Par exemple, il peut s'agir de billes revêtues, notamment par absorption ou par lien chimique, desdits éléments de capture. Ladite phase solide pouvant être ajoutée dans ledit récipient soit au début de la culture soit en cours de culture. Parmi les éléments physiquement indépendants, il faut citer également les éléments solidaires d'une pièce de préhension, notamment un couvercle, qui peuvent ainsi être transférés dans un autre récipient après l'étape de culture.

Dans un mode de réalisation illustrant l'invention, la population cellulaire à étudier, éventuellement diluée dans un milieu de culture adéquat contenant facultativement des agents activateurs, c'est-à-dire stimulateurs de la production, est distribuée quantitativement sur un support de culture au niveau duquel se fera la production du ou des médiateurs solubles à mesurer, ce support comprenant d'emblée un élément essentiel du futur dosage sous forme d'anticorps ou d'antigènes fixés de manière adéquate sur une phase solide qui peut être le support. Au moment choisi, la culture est interrompue par un lavage approprié des cellules et le dosage des médiateurs produits est directement achevé sur le support sans transfert supplémentaire du milieu de culture.

Ainsi, par exemple, dans un mode de réalisation particulier, on dilue du sang complet fraîchement prélevé, par exemple au 1/5ème ou au 1/10ème, dans un milieu de culture contenant facultativement un agent activateur tel que de la PHA, des LPS, ou autres, et on distribue cette suspension cellulaire sur une plaque de microtitration sur laquelle sont déjà fixés des anticorps nécessaires aux dosages. La plaque est mise à incuber dans des conditions standardisées (température, humidité, pression partielle en O₂ et CO₂, etc...) pendant une durée choisie, qui peut être par exemple de I heure à 3 jours. A la fin de cette période, la plaque est lavée à l'aide d'une solution adéquate qui permet l'élimination des cellules et la préservation des anticorps fixés à la plaque ainsi que des antigènes (par exemple cytokines) produites pendant la culture. Un anticorps traceur, c'est-à-dire marqué par exemple par un enzyme ou un isotope, est ajouté dans les puits, ou bien ce traceur peut également être d'emblée présent dans le milieu de culture initial. La révélation du traceur et donc du médiateur à mesurer est alors directement réalisée dans la plaque de microtitration selon les méthodes conventionnelles.

Les traceurs, c'est-à-dire par exemple des anticorps marqués par un enzyme ou un isotope radioactif, peuvent être ajoutés dans les puits de culture soit au moment de la répartition initiale des cellules dans le milieu (le traceur pouvant être d'emblée incorporé à ce milieu), soit à tout autre moment plus tardif de la culture, voire après lavage des cellules.

La quantité produite de la molécule recherchée peut être déterminée par extrapolation par rapport à une courbe standard, réalisée à partir d'échantillons standards incubés dans les mêmes conditions, soit par une mesure directe de la radioactivité dans le cas d'un traceur radioactif, soit après réaction avec le révélateur enzymatique dans le cas d'un traceur enzymatique, soit par toute autre méthode conventionnelle.

Toute molécule dosable par méthode immunologique et produite par des cellules in vitro peut être analysée selon l'invention. On peut citer notamment les cytokines, monokines, lymphokines, les facteurs de croissance, facteurs de différenciation, hormones, neuropeptides, kinines, histamines, protéines cationiques des éosinophiles, protéines basiques majeures ou autres marqueurs de dégranulation, facteurs de coagulation, récepteurs solubles, molécules d'adhésion et des matrices extracellulaires, ainsi que des molécules d'origine infectieuse dont la production est assurée par la culture cellulaire, par exemple des particules virales.

Il peut s'agir, dans le cas de particules infectieuses, de virus, d'éléments viraux antigéniques et/ou d'autres germes intracellulaires et/ou de leurs éléments antigéniques.

A titre de cellules, le sang convient particulièrement bien au procédé selon l'invention de par le fait qu'il s'agit d'un tissu dans lequel les éléments cellulaires sont circulants. La méthode, selon l'invention, peut cependant être étendue à d'autres types cellulaires que les cellules du sang.

Des cellules de toutes origines, dans leur milieu naturel ou non, peuvent être utilisées selon l'invention. On peut citer par exemple le sang complet dilué ou non, ou des cellules sanguines isolées et purifiées; des cellules provenant d'autres fluides biologiques tels que les liquides synovial, pleural, péritonéal, céphalo-rachidien, ou autres; des cellules isolées à partir d'un tissu solide de foie, peau, glandes, tumeurs, ou autres; des lignées cellulaires humaines ou animales; des cellules extraites d'un tissu solide, notamment les hépatocytes ou cellules de Kupfer extraites d'une carotte hépatique, des fragments de tissu solide, notamment des fragments de tissu épithélial, ou fragments de tumeurs.

On peut, selon le procédé de l'invention, mesurer la production spontanée (c'est-à-dire non stimulée) du paramètre à doser, ou sa production stimulée. De très nombreux activateurs sont possibles selon les cellules et les paramètres étudiés et la signification attendue des résultats. On peut citer par exemple:
- des activateurs polyclonaux non spécifiques agissant sur les systèmes d'activation intracellulaire, notamment le phorbol ester (TPA ou autre), des ionophores (A23185, ionomycine, ou autres.
- des activateurs polyclonaux sélectifs de certaines populations cellulaires, par exemple des lectines, la PHA, la Con-A (concanavaline), PKM; LPS (lipopolysacharides), des endotoxines.
- des activateurs spécifiques de type anticorps, notamment anti CD3, anti Ig M, anti-récepteurs ou anti-molécules membranaires impliquées dans l'activation cellulaire.
- des activateurs spécifiques de type antigènes purifiés ou non, synthétiques ou naturels, par exemple les autoantigènes des pathologies auto-immunes, les antigènes d'origine bactérienne, virale ou parasitaire, les allergènes, les superantigènes.
- des ligands naturels ou synthétiques, y compris des médicaments, voire des cytokines, de récepteurs cellulaires pour études pharmacologiques.

On peut avoir recours également, dans le procédé de l'invention, à des inhibiteurs, par exemple des anticorps neutralisant la liaison cytokine - récepteur ou cytokine - protéine de liaison, pour amplifier la détection de la cytokine.

On peut également prévoir comme additif au milieu de culture des agents agissant sur des médiateurs non dosés, ou ces médiateurs eux-mêmes, afin d'interférer volontairement avec la régulation de la production du médiateur à doser. Par exemple la neutralisation de l'IFN Y par un Anticorps neutralisant permet d'amplifier la production d'IL4, libérée de cette contrainte inhibitrice. L'addition d'IL4 exogène peut stimuler la production du CD23 que l'on désire mesurer.

Il importe que le milieu et les conditions de culture soient standardisés pour chaque application afin d'obtenir des résultats comparables et reproductibles. Dans la majorité des exemples qui suivront, on peut utiliser le RPMI 1640 comme milieu de culture ou de dilution de base et des incubations à 37° en atmosphère saturée en eau pour des périodes variant de 1 à 72 heures. De très nombreuses variantes sont évidemment possibles en ce qui concerne le milieu et les conditions de culture, notamment selon le type cellulaire étudié.

Le lavage est une étape importante du procédé selon l'invention, car il convient d'éliminer suffisamment les cellules qui adhérent le cas échéant au support commun de la culture et du dosage, sans dégrader les éléments du dosage, afin d'éviter toute interférence avec les étapes finales du dosage telles que par exemple une réaction enzymatique ou une mesure d'absorbance. Les cellules qui adhèrent au fond des puits, par exemple, sont susceptibles d'activer le substrat de l'enzyme et de donner un faux signal positif. Le lavage doit permettre le détachement complet des cellules sans détériorer les complexes antigènes-anticorps.

Les "agents de détachement" cellulaires, qui sont de préférence ajoutés au milieu de lavage, comprennent notamment:
- des agents chelatant tels que EDTA, EGTA;
- des enzymes protéolytiques de type trypsine, dispase, ou autres;
- des détergents de type Tween, Triton, ou autres;
- des inhibiteurs d'adhésion de type polysaccharidiques, notamment héparines, ou peptidiques (par exemple, comprenant une séquence RGDS ou analogue);
- ou un mélange de ces différents facteurs.

Dans un mode préféré de réalisation de l'invention, les solutions de lavage mises au point contiennent des enzymes protéolytiques, par exemple de la Trypsine. Dans les conditions d'utilisation, ces enzymes protéolytiques ne détruisent pas les éléments du dosage (complexesAg-Ac) fixés sur la phase solide.

Les conditions de lavage peuvent être manuelles ou, de préférence, automatiques et répétées de manière standardisée.

Selon l'invention, le dosage est un dosage immunologique sur phase solide, la phase solide étant présente avec la culture cellulaire au moment de la production du paramètre à étudier.

Pour autant qu'il soit sur phase solide, le dosage peut obéir aux différents principes bien connus de l'homme de l'art, par exemple tests par compétition ou en sandwich. Les méthodes de révélation peuvent être de toutes natures connues de l'homme de l'art, enzymatiques, isotopiques, basées sur la luminescence, sur la fluorescence, sur la diffraction néphélométrique, etc...

Les méthodes de dosage qui peuvent être utilisées sont en particulier les:
- méthodes sandwiches de type ELISA utilisant des anticorps traceurs marqués à la peroxidase ou, bien entendu avec d'autres enzymes que la peroxidase, ribonucléase, phosphase alcaline, acétylcholinesterase, et autres;
- méthodes compétitives de type EIA utilisant des antigènes ou éventuellement des anticorps antiidiotypiques traceurs (cf. ELISA), pour autant que l'anticorps de capture soit sur une phase solide de type tube/plaque/bille revêtue d'anticorps;
- méthodes sandwiches de type IRMA utilisant des anticorps traceurs radioactifs (¹²⁵I, ¹³¹I, ³H,...);
- méthodes compétitives de type RIA utilisant des antigènes traceurs (cf. IRMA), pour autant que l'anticorps de capture soit sur une phase solide de type tube/plaque/bille revêtue d'anticorps;
- toutes méthodes d'immunodosages de type sandwiche ou compétition basées sur d'autres types de traceurs (luminescence, fluorescence...) et plus généralement
- toutes autres méthodes d'immunodosages sur phase solide basées sur d'autres principes que le sandwiche ou la compétition, en particulier l'agglutination.

Les éléments de captures, fixés à la phase solide, peuvent être des anticorps, ou des antigènes, ou des récepteurs spécifiques de la molécule à doser, ou tout autre ligand, notamment ligand de récepteur soluble, spécifique de la molécule à doser. Il en est de même des traceurs utilisés.

Les anticorps qui peuvent être utilisés peuvent être monoclonaux, oligoclonaux (c'est-à-dire qu'on utilise plusieurs anticorps monoclonaux reconnaissant des épitopes différents du même antigène) ou polyclonaux, entiers ou sous forme de fragments. Ils sont sélectionnés pour la qualité du dosage et sélectionnés ou non pour leurs propriétés biologiques concernant la production du paramètre à doser, sa stabilité, sa préservation, sa capture, son démasquage vis-à-vis des molécules liantes.

En effet, de préférence, les anticorps seront sélectionnés et choisis non seulement pour leurs qualités "statiques" dans le dosage mais aussi pour l'influence qu'ils peuvent exercer sur la production "dynamique" du médiateur (par exemple en interférant avec les boucles de régulation de la production), sur sa stabilité (par exemple en empêchant la dégradation du médiateur), sur sa disponibilité (par exemple en empêchant la recaptation et consommation du médiateur par des cellules ou en empêchant son masquage par des éléments solubles capables de le complexer), etc ...

Dans le cadre de certaines applications particulières, notamment pour le dosage des cytokines produites par les cellules activées du sang complet, il peut être préférable que les anticorps participant au dosage n'interfèrent pas avec les activités paracrines et autocrines des cytokines dosées qui, le cas échéant, influencent les processus d'activation et de production de ces cytokines. En effet, si c'était le cas, comme ces anticorps sont présents au moment de la stimulation, la production finale de certaines cytokines pourrait être modifiée. Cela implique que les anticorps utilisés pour les dosages ne modifient pas l'activité biologique des cytokines dosées (anticorps neutres), c'est-à-dire que ces anticorps doivent de préférence reconnaître les cytokines par des épitopes distincts du site de liaison de la cytokine à son substrat (récepteur cellulaire).

Cette notion peut être illustrée par deux exemples:
1°) L'IL2
   L'IL2 a une action autocrine positive sur sa propre production en se liant sur un récepteur spécifique présent sur la cellule T productrice d'IL2. Si l'un des anticorps participant au dosage in situ bloquait la liaison de l'IL2 à son récepteur, la production finale d'IL2 serait diminuée. Par contre, la simple fixation de l'IL2 sur la phase solide ne supprime pas son activité biologique.
2°) L'IL1
   L'IL1 produite par les monocytes potentialise l'activation des cellules T par la PHA. Les cellules T en retour produisent de l'IL4 qui inhibe la production d'ILI par les monocytes. Si l'un des anticorps participant au dosage dynamique de l'IL1 bloquait son activité biologique sur les cellules T, la production d'IL4 serait diminuée et le retrocontrôle négatif sur la production d'IL1 se ferait moins bien avec comme résultat une production exagérée d'IL1.

On pourra étudier, selon l'invention, la production d'une cytokine particulière ou d'un panel de cytokines prédéterminé:
- soit en utilisant des plaques de microtitration "multicoatées" (c'est-à-dire revêtues de plusieurs anticorps de spécificités différentes) et en assurant la spécificité du dosage par le choix d'un traceur adéquat;
- soit en utilisant une plaque de microtitration revêtue par un anticorps ou une combinaison d'anticorps donnant la spécificité, le traceur pouvant dès lors être spécifique d'une seule cytokine mais aussi comprendre un mélange d'anticorps dirigés contre les différentes cytokines d'intérêt.

Plus précisément, dans un mode de réalisation, la présente invention a donc pour objet un procédé dans lequel on dose plusieurs dites molécules caractérisé en ce que:
a) on effectue simultanément la culture cellulaire dans autant de groupes de récipients que de molécules à doser, récipients tels que les puits d'une plaque de microtitration;
b) sur les phases solides incorporées dans chaque récipient sont fixées plusieurs familles d'anticorps de capture, chacune reconnaissant une desdites molécules, c'est-à-dire un mélange d'anticorps de spécificités différentes reconnaissant l'ensemble des molécules à doser.
c) on dose chaque molécule après incubation dans chaque récipient avec un traceur différent spécifique de chaque molécule correspondante.

Dans un autre mode de réalisation, la présente invention a pour objet un procédé dans lequel on dose plusieurs dites molécules caractérisé en ce que:
a) on effectue simultanément la culture cellulaire dans autant de groupes de récipients que molécules à doser, tels que des puits de plaque de microtitration, et
b) sur la phase solide incorporée dans chaque groupe de récipient sont fixés des anticorps de spécificité prédéterminée reconnaissant dans chaque groupe de récipient une molécule éventuellement différente, et
c) on dose chaque molécule après incubation de chaque récipient avec un traceur spécifique de la molécule correspondante ou un traceur commun qui est un mélange de traceurs spécifiques des différentes molécules, la spécificité étant assurée par les anticorps de capture.

Comme on l'a vu, de préférence, les anticorps participant au dosage à titre de traceur ou d'élément de capture de la molécule à doser fixés à la phase solide, seront sélectionnés de manière telle qu'ils reconnaissent la molécule à doser par des épitopes n'affectant pas la production de ladite molécule et/ou son activité biologique.

Ainsi, si l'on considère l'exemple d'une plaque de titration comprenant 12 à 96 puits amovibles ou non, cette plaque peut être prévue pour doser la production d'un seul paramètre dans différentes conditions de culture (avec ou sans activateurs, pendant un temps court ou long, etc...), correspondant aux différents puits. La plaque peut également être conçue pour mesurer différents paramètres (par exemple a puits pour l'IL6, b puits pour le TNF, c puits pour l'IL1, etc...). Dans ce cas, comme on l'a mentionné, deux approches sont possibles:
- soit on utilise un support sur lequel des anticorps de différentes spécificité (anti IL6, anti TNF, anti IL1, ...) sont fixés, la spécificité du dosage étant alors assurée par l'anticorps traceur (anti IL6 ou anti TNF par exemple). L'avantage pour le manipulateur est de pouvoir disposer librement ses différentes conditions sur le support (ici la plaque) et de choisir le paramètre étudié par le traceur ajouté ultérieurement ("Plaques multicoatées");
- soit on utilise un support où les anticorps présents n'ont qu'une seule spécificité moléculaire (anti IL6 ou anti TNF par exemple), mais on dispose d'un traceur comprenant un mélange d'anticorps marqués de différentes spécificités (anti IL6, anti TNF, etc, ...). Dans ce cas, la spécificité du dosage est assurée par le support et est donc préalablement fixée. L'avantage pour le manipulateur est de disposer d'un traceur commun qu'il peut ajouter dans le milieu de culture avant même la distribution sur le support, ou ultérieurement ("Traceur multiparamètres").

Plusieurs cytokines qui, après un temps de culture, sont produites à des taux directement mesurables, peuvent être produites à des taux largement supérieurs à la capacité maximum de dosage de l'immunodosage après un temps plus long de culture (exemple IL6 et IFNg). Dans un dosage classique, les échantillons trop élevés sont dilués suffisamment pour pouvoir être dosés. Dans le cas du dosage in situ, selon l'invention, la production de cytokines se faisant au niveau du site de dosage, une telle dilution n'est pas possible. Afin de contourner cette limitation du dosage in situ, on peut, selon l'invention, réaliser à la fois un dosage sensible et un dosage désensibilisé au cours d'une seule expérience.

Dans ce cas, on a recours aux éléments suivants:
- une plaque de microtitration revêtue avec des anticorps spécifiques de la ou des molécules que l'on désire mesurer,
- un milieu de culture A pouvant facultativement contenir d'emblée des activateurs et/ou un anticorps traceur spécifique de la ou des molécules que l'on désire mesurer,
- un milieu de culture B pouvant facultativement contenir d'emblée des activateurs et/ou un anticorps traceur spécifique de la ou des molécules que l'on désire mesurer et une quantité déterminée d'anticorps froids pour désensibiliser le dosage,
- des solutions d'échantillons standard,
- les solutions de lavage,
- le révélateur enzymatique et la solution d'arrêt dans le cas d'un traceur enzymatique,
- des activateurs appropriés, si ceux-ci ne sont pas incorporés dans le milieu de culture,
- une solution A d'anticorps traceurs appropriés, si ceux-ci ne sont pas incorporés dans le milieu de culture A,
- une solution B d'anticorps traceurs appropriés et une quantité déterminée d'anticorps froids pour désensibiliser le dosage si ces éléments ne sont pas incorporés dans le milieu de culture B.

Les échantillons cellulaires à tester (éventuellement le sang complet) sont cultivés simultanément dans le milieu de culture A (ou en présence de solution traceur A) et dans le milieu de culture B (ou en présence de solution traceur B). Pour des concentrations de cytokines produites dans le milieu A (en présence de solution traceur A), entrant dans le domaine de concentration de la gamme standard, la valeur obtenue dans le milieu A (en présence de solution traceur A) est lue directement sur la gamme standard. Pour des concentrations de cytokines produites dans le milieu A (en présence de solution traceur A), supérieures au dernier point standard, la valeur correspondante obtenue dans le milieu B (en présence de solution traceur B) est lue directement sur la gamme standard et multipliée par un facteur de correction prédéterminé.
C'est également un objet de l'invention d'avoir montré qu'un tel procédé de désensibilisation et de définition d'un facteur de correction est possible.

La présente invention a également pour objet un procédé tel que décrit précédemment et caractérisé par une désensibilisation du dosage obtenue par un mélange de l'Anticorps traceur spécifique de la molécule à doser avec un anticorps non marqué de même spécificité, désensibilisation qui permet grâce à un facteur correctif prédéterminé d'étendre la gamme de dosage vers les valeurs élevées.

La présente invention a également pour objet un procédé caractérisé en ce que les échantillons cellulaires à doser sont cultivés simultanément dans un premier milieu de culture comprenant un anticorps traceur spécifique de la molécule à doser et dans un deuxième milieu de culture comprenant un anticorps traceur spécifique de la molécule à doser et un anticorps non marqué de même spécificité pour désensibiliser le dosage et permettre la lecture sur une nouvelle échelle de concentration par rapport aux standards..

De façon non exhaustive, on peut citer les avantages suivants du procédé de l'invention:
1. Grâce au processus d'intégration culture/dosage, on aboutit à une très grande simplicité de réalisation et rapidité.
2. Le procédé ne nécessite pas le matériel de séparation cellulaire et cellules/surnageant indispensable selon les procédés conventionnels (par exemple centrifugeuses ...).
3. Les manipulations des cellules sont très réduites d'où une réduction des causes d'activation ou de sélection involontaires des cellules faussant l'interprétation des résultats.
4. On observe une très grande reproductibilité des résultats.
5. Des possibilités d'automatisation de tout ou partie de la procédure sont beaucoup plus grandes, notamment suite à la suppression des étapes de récolte de surnageants, de stockage et de redistribution selon les procédés multiétapes conventionnels.
6. Suite à la simplicité, la faible manipulation cellulaire et les possibilités d'automation, le risque de contamination infectieuse pour le manipulateur (Cfr hépatites, sida,...) est très faible.
7. La production du médiateur et les premières étapes du dosage sont réalisables dans l'environnement naturel (ou sélectionné) des cellules, enfin et surtout
8. On procure aux laboratoires de biologie clinique un accès plus facile et plus routinier à un nouveau domaine d'exploration clinique, c'est-à-dire l'étude des capacités de production.

L'invention a également pour objet un format technologique ainsi que des "kits" standardisés pour l'évaluation de la production de divers paramètres, utilisés pour mettre en oeuvre le procédé de l'invention.

Ces kits comprennent le(s) récipient-support(s) commun(s), selon l'invention, à l'étape de culture et d'initiation du dosage ainsi que éventuellement les milieux de culture, milieux de lavage, activateurs, révélateurs et échantillons standards du dosage, et autres réactifs nécessaires au dosage selon l'invention. Plus précisément, ces kits comportent:
- ledit récipient-support de culture cellulaire servant de récipient de dosage, ladite phase solide sur laquelle sont fixés les éléments de capture de l'immunodosage, étant incorporée ou incorporable audit récipient (c'est-à-dire physiquement dépendant ou indépendant du récipient) ;
- ainsi tous les éléments du dosage, y compris le traceur, des échantillons standards, ainsi que des milieux de culture, de dilution cellulaire, de lavage et des agents stimulateurs ou régulateurs de production des paramètres à mesurer.

Comme on l'a vu, le récipient-support solide sera de préférence sous forme de puits assemblables sous forme d'une plaque de titration ou sous forme de tubes qui pourront également être assemblés pour la commodité de l'utilisateur et l'adaptation aux instruments standardisés (par exemple laveur). D'autres supports pour des applications particulières (boîtes de petri, lames de verre compartimentalisées, etc...) voire une dissociation physique entre le support de la culture et le support du dosage pour autant qu'ils soient destinés à être rassemblés lors de la production des paramètres à doser, par exemple des billes recouvertes d'Anticorps sont également appropriés.

Selon une variante, le traceur et/ou l'agent d'activation (par exemple PHA, LPS, ConA,...) peut être incorporé sous forme séchée (par évaporation ou lyophilisation) dans lesdits récipients, notamment dans des puits de la microplaque revêtus des anticorps participant au dosage. Dans ce cas particulier, il suffit d'ajouter le sang complet fraîchement prélevé dans les micropuits en présence d'un volume adéquat de milieu de culture.

Selon une variante, les échantillons standards peuvent être incorporés sous forme séchée (par évaporation ou lyophilisation) dans lesdits récipients, notamment dans les puits de la microplaque revêtus des anticorps participant au dosage. Dans ce cas particulier, il suffit de réhydrater les standards avec de l'eau et d'ajouter le même volume de milieu de culture que pour l'échantillon.

Le procédé et les formats technologiques selon l'invention peuvent avoir de multiples applications dont certaines seront illustrées en détail dans les exemples. On peut citer, de manière non exhaustive, les différentes applications suivantes:

### 1. Evaluation de la capacité de production des cytokines par les cellules du sang.

On utilise du sang complet dilué, mais on peut éventuellement réaliser une séparation cellulaire préalable. On peut étudier la production spontanée de cytokines, ainsi que la production stimulée. On peut analyser la quantité de cytokine produite, la cinétique de production et le pouvoir stimulant de différents activateurs à différentes concentrations, et autres. Ce type d'étude permet d'évaluer notamment:
- des déficits de productions, liés à un déficit congénital ou acquis, à un épuisement cellulaire dans des conditions de fortes stimulations in vivo, à l'action d'un toxique ou d'un médicament immunosuppresseur dont on évalue l'activité, à une dysrégulation dans le cadre d'une pathologie immunitaire, à un bouleversement des répartitions cellulaires lié à un état tumoral ou autre.
- des excès de production, correspondant à un état d'activation anormal des cellules productrices en relation avec un état pathologique, une transplantation d'organe, un état infectieux, une maladie auto-immunitaire, un cancer, et autres.
Ces analyses conduisent à des informations de valeur diagnostique (par exemple déficit immunitaire congénital ou acquis; rejet d'organe,...) ou pronostique (par exemple risque d'une récidive d'une agression auto-immune (sclérose en plaque, diabète,...); risque de présenter une complication (Choc septique, ...); ainsi que des informations sur l'efficacité thérapeutique (par exemple traitement immunosuppresseur). Ces informations influencent donc les décisions et actes médicaux et rentrent donc parfaitement dans la fonction de la biologie clinique.

### 2. Tests de provocation in vitro.

La sensibilité aux allergènes est déterminée par le dosage des Ig E plasmatiques spécifiques ou par des tests de provocation in vivo. Ces deux méthodes présentent des inconvénients majeurs et ne sont que d'une fiabilité très relative.
Selon l'invention, les cellules (c'est-à-dire par exemple le sang complet dilué) d'un patient allergique sont incubées sur un support servant au dosage d'un médiateur de l'allergie, par exemple un médiateur issu des basophiles ou des éosinophiles (par exemple protéines cationiques des éosinophiles, protéine majeure basique et autres marqueurs de dégranulation, ECFA, NCFA, ...). L'agent stimulateur est ici le ou les allergènes incriminés.
L'évaluation de la production de médiateurs de l'allergie en réponse à l'allergène in vitro correspond à un "test de provocation in vitro" qui, dans le cadre du procédé et du format selon l'invention, devient accessible à des laboratoires non spécialisés.

### 3. Tests de compétence immunologique spécifiques.

Les cellules, purifiées ou non, d'un patient sont mises en présence d'antigènes spécifiques, et la capacité de réponse à cet antigène est évaluée par la mesure de production de cytokines selon l'invention. On peut citer à titre d'illustration deux exemples:
a. Les cellules d'un patient sont incubées en présence d'un autoantigène présumé, par exemple, la "Myelin basic protein" ou ses fragments antigéniques dans la sclérose en plaque. Une réponse anormale à cet autoantigène est évaluée à travers la mesure de la production de cytokines dans un format technologique selon l'invention.
b. Une culture mixte lymphocytaire est réalisée directement sur un support selon l'invention. La réactivité est évaluée par la mesure de la production de cytokines à différents temps d'incubation (test de compatibilité in vitro).

### 4. Investigations pharmacologiques in vitro.

L'influence de médicaments, administrés au malade ou bien présents dans le milieu d'incubation in vitro, sur la production de médiateurs peut être facilement étudiée en utilisant le format selon l'invention. Ceci permet d'effectuer un suivi thérapeutique (par exemple puissance de l'immunosupression) ou des prédictions de réponses.

### 5. Biodosage et immunodosage intégrés.

Les dosages biologiques visent à enregistrer une réponse cellulaire à unstimulus donné. Les dosages biologiques présentent parfois certains avantages sur d'autres méthodes de dosage (par exemple immunologiques) et sont parfois sans alternatives. La réponse cellulaire peut être mesurée de diverses façons, par exemple une réponse proliférative par incorporation de thymidine, une réponse différenciative par coloration. La production d'une substance peut être la réponse cellulaire mesurée dans le bioassay. Dans ce cas, le test se fait en trois étapes: le bioassay proprement dit, la récolte (et stockage) du surnageant, et le dosage du surnageant à l'aide d'un immunodosage. Le procédé selon l'invention permet d'intégrer ces différentes étapes (voir exemple).
D'une manière générale, la cellule cible (par exemple une lignée cellulaire) est mise en présence de l'agent stimulant sur un support qui porte déjà les éléments de dosage du paramètre d'évaluation de la réponse cellulaire. Après l'incubation, les cellules sont lavées et la révélation du dosage réalisée directement sur le support sans transferts supplémentaires.

D'autres caractéristiques et avantages de la présente invention apparaîtront à la lumière des exemples qui vont suivre.
- Les figures 1 à 6 représentent les résultats des expériences de l'exemple 1 relatives au Format unicytokine.
- Les figures 7 à 12 représentent les résultats des expériences de l'exemple 2 relatives au Format multicytokines.
- La figure 13 représente les résultats de l'expérience de stabilité de l'exemple 3.
- Les figures 14 à 16 représentent les résultats des expériences de lavage de l'exemple 4.
- Les figures 17 et 18 représentent les résultats de dosage de l'IFNg par dosage in situ et dosage classique de l'exemple 5.

### EXEMPLE 1 : FORMAT DE DOSAGE UNICYTOKINE

### 1) matériel fourni sous forme de kit de dosage in situ monocytokine

- une plaque de microtitration revêtue avec des anticorps spécifiques de la cytokine que l'on désire mesurer.
- une fiole contenant une solution de milieux de culture + 5 ug/ml de PHA et 25 ug/ml de LPS + l'anticorps traceur (solution A) ou sans PHA et LPS (solution A')
- un jeu de fioles contenant des solutions standards de la cytokine à doser (solution standards 1 à n).
- une fiole de solution de lavage concentrée (solution B).
- une fiole de solution de lavage intermédiaire contenant de la trypsine (solution C).
- une fiole de solution concentrée de substrat de l'enzyme avec lequel l'anticorps traceur est marqué à la peroxydase (solution D),
- une fiole de solution tampon pour diluer le substrat (solution E).
- une fiole d'H2S04 pour bloquer la réaction enzymatique (solution F).

### 2) procédure

- délimiter la partie de la plaque qui va servir aux standards et aux échantillons à tester.
- remplir tous les puits avec 200 ul de la solution A ou A'.
- pour la gamme standard, ajouter 25 ul des différents points standards dans les puits correspondants.
- pour les échantillons, ajouté 25 ul de sang total par puits.
- incuber pendant différents temps 37 ° C en atmosphère saturée en eau.
- laver les plaques comme suit :
   . aspirer-laver 4 fois avec la solution B diluée
   . remplir les puits avec 200 ul de la solution C
   . aspirer-laver 3 fois avec la solution B diluée
- ajouter 200 ul par puits de la solution substrat (solution D diluée dans E)
- incuber 15 minutes sous agitation à température ambiante.
- ajouter 50 ul de la solution bloquante (solution F).
- lire la plaque dans un spectrophotomètre et extrapoler la concentration de la cytokine produite par rapport à la courbe standard.

### 3) expérience

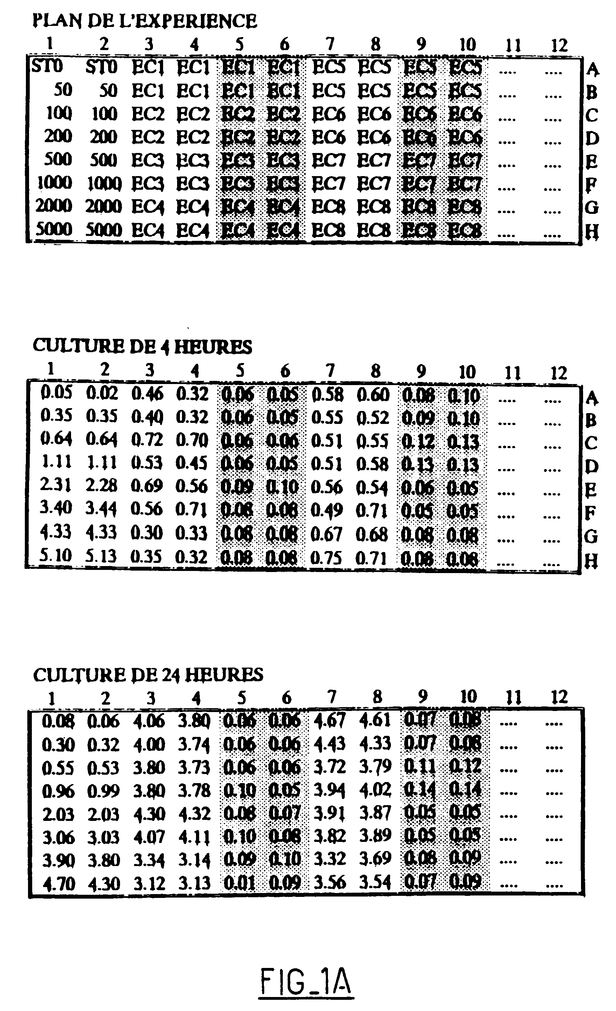

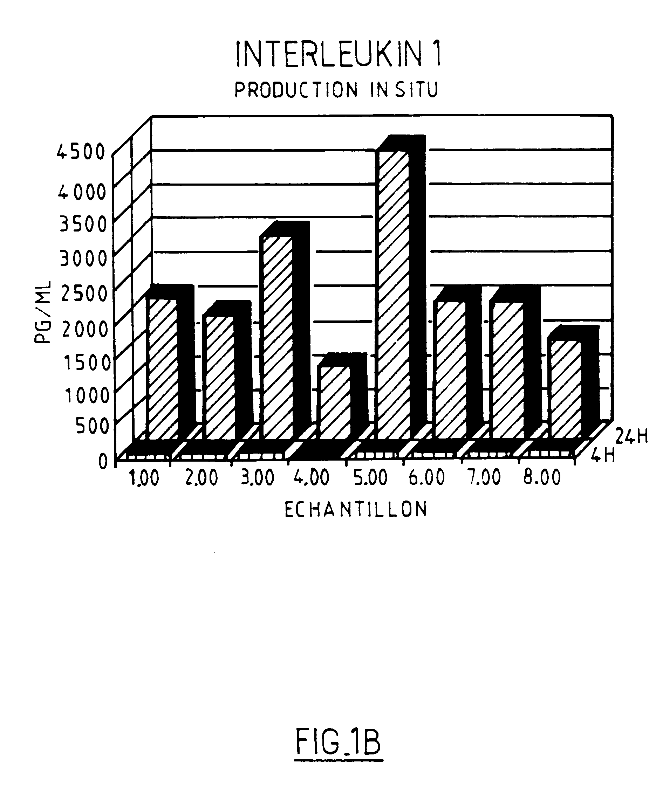

- dosage des cytokines IL1B, IL6, TNFa , IL2, IFNa , et GM-CSF par la méthode décrite suivant le format technique unicytokine décrit et effectué sur 8 échantillons de sang complet.
- pour chaque cytokine sont montrés sur les figures 1 à 6 :
   . le plan de l'expérience représenté par un schéma de plaque ou sont localisés les échantillons standards (ST) exprimés en picogrammes par millilitre pour IL1, IL6,TNFa et GM-CSF et en unites internationales pour IL2 et IFNg ainsi que les échantillons (EC 1 à 8).
   . un schéma de plaque représentant les densités optiques lues après 4 heures de culture.
   . un schéma de plaque représentant les densités optiques lues après 24 heures de culture.
   . les densités optiques représentent l'intégration de deux lectures, l'une à 450 nm. et l'autre à 490 nm.
   . une partie des plaques est revêtue avec des anticorps spécifiques de la cytokine recherchée. Les valeurs lues sur cette partie des plaques représentent le signal spécifique qui est proportionnel à la quantité de cytokine produite.
   . une partie des plaques est revêtue avec des anticorps non spécifiques (zones hachurées). Les valeurs lues sur cette partie des plaques représentent le bruit de fond de la méthode.
   . un graphique représentant les concentrations en cytokines produites extrapolées par rapport à la courbe standard après 4 et 24 heures de culture.
   . chaque échantillon est mesuré en quadrupliqué sur l'anticorps spécifique et en quadrupliqué sur l'anticorps non spécifique.

### 4) conclusions :

a) Dans les conditions du format on obtient des courbes standard d'allures entièrement satisfaisantes et cela aussi bien après 4 ou 24 heures de culture.
b) dans ces conditions on observe des productions significatives d'ILl, d'IL6, et de TNFa après 4 heures de culture. Ces productions sont considérablement augmentées après 24 heures de culture.
c) dans ces mêmes conditions des productions significatives d'IL2, d'IFNg et de GM-CSF peuvent être observées après 24 heures de culture.
d) dans tous les cas le bruit de fond est suffisamment bas : < 0.2 unités de densité optique.
e) dans tous les cas la reproductibilité des quadruplets est remarquable.

### EXEMPLE 2 : FORMAT DE DOSAGE MULTICYTOKINES

### 2-1 : FORMAT SELON LE PRINCIPE DU MULTITRACEUR

### A) Matériel fourni et procédure :

Ils sont identiques à celui de l'exemple 1 sauf :
- solution A contenant un traceur multicytokine (ex:anticorps anti IL1 + anticorps anti IL6 + anticorps anti TNFa).
- solutions standard multicytokines IL1 + IL6 + TNFa

### B) Plan de l'expérience :

1) rangées 1 et 2 : puits revêtus avec anticorps unispécifiques anti IL1 (ou anti IL6 ou anti TNF) + standard multicytokines IL1 + IL6 + TNF (0, 100, 500, et 2000 pg/ml) ou échantillons (EC1 et EC2) + traceur multicytokine anti IL1 + anti IL6 + anti TNF.
2) rangées 3 et 4 : puits revêtus avec anticorps unispécifiques anti IL1 (ou anti IL6 ou anti TNF) + standard unicytokines IL1 (0, 100, 500, et 2000 pg/ml) ou échantillons (EC1 et EC2) + traceur multicytokine anti IL1 + anti IL6 + anti TNFa.
3) rangées 5 et 6 : puits revêtus avec anticorps unispécifiques anti IL1 (ou anti IL6 ou anti TNF) + standard unicytokines IL6 (0, 100, 500, et 2000 pg/ml) ou échantillons (EC1 et EC2) + traceur multicytokine anti IL1 + anti IL6 + anti TNFa.
4) rangées 7 et 8 : puits revêtus avec anticorps unispécifiques anti IL1 (ou anti IL6 ou anti TNF) + standard unicytokines TNF (0, 100, 500, et 2000 pg/ml) ou échantillons (EC1 et EC2) + traceur multicytokine anti IL1 + anti IL6 + anti TNFa.
5) rangées 9 et 10 : puits revêtus avec anticorps unispécifiques anti IL1 (ou anti IL6 ou anti TNFa) + standard unicytokine (IL1 ou IL6 ou TNF) ou échantillons (EC1 et EC2) + traceur unicytokine (anti IL1 ou anti IL6 ou anti TNFa).
6) rangée 11 : puits revêtus avec anticorps non spécifiques + échantillons (EC1 et EC2) + traceur unicytokine (anti IL1 ou anti IL6 ou anti TNFa).
7) rangée 12 : puits revêtus avec anticorps non spécifiques + échantillons (EC1 et EC2) + traceur multicytokine anti IL1 + anti IL6 + anti TNFa).

Chaque dosage d'intérêt est représenté par une feuille d'expérience représentant :
- le plan de l'expérience
- les densités optiques obtenues après 4 heures de culture
- les densités optiques obtenues après 24 heures de culture.

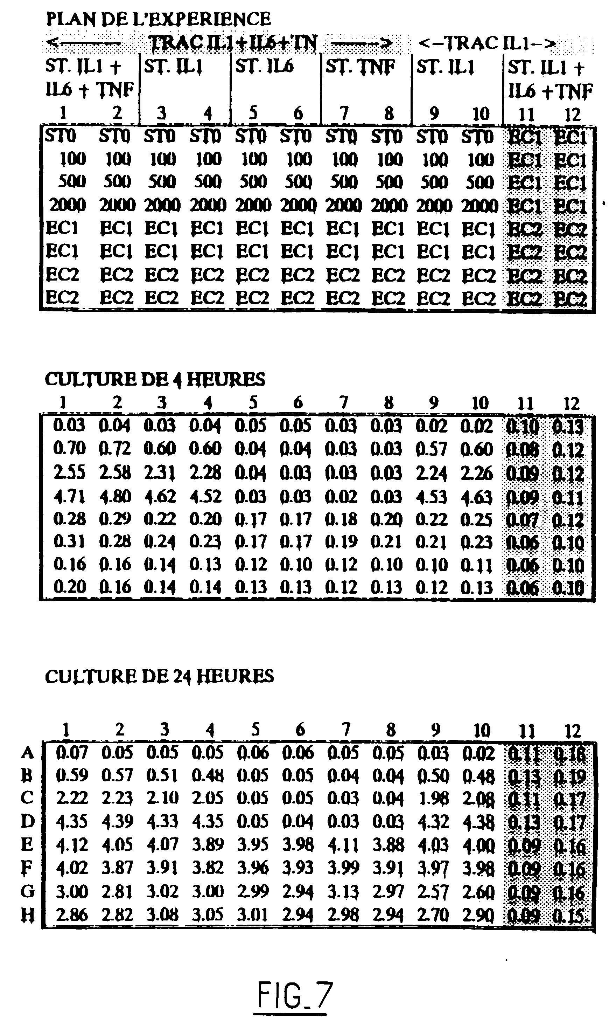

Les résultats sont présentés aux figures 7 à 9.

### 2-2 : FORMAT SELON LE PRINCIPE DU MULTIREVETEMENT

### A) Matériel fourni et procédure :

C.f. format unicytokine sauf :
- plaques multicoatées avec une mixture d'anticorps de différentes spécificités : anti IL1 + anti IL6 + anti TNF.
- solutions standard multicytokines IL1 + IL6 + TNF.

### B) Plan de l'expérience :

1) rangées 1 et 2 : puits revêtus avec anticorps plurispécifiques (anti IL1 + anti IL6 + anti TNF) + standard multicytokines IL1 + IL6 + TNF (0, 100, 500,et 2000 pg /ml) ou échantillons (EC1 et EC2) + traceur unicytokine (anti IL1 ou anti IL6 ou anti TNF).
2) rangées 3 et 4 : puits revêtus avec anticorps plurispécifiques (anti IL1 + anti IL6 + anti TNF) + standard unicytokines IL1 (0, 100, 500, et 2000 pg/ml) ou échantillons (EC1 et EC2) + traceur unicytokine (anti IL1 ou anti IL6 ou anti TNF)
3) rangées 5 et 6 : puits revêtus avec anticorps plurispécifiques (anti IL1 + anti IL6 + anti TNF) + standard unicytokines IL6 (0, 100, 500, et 2000 pg/ml) ou échantillons (EC1 et EC2) + traceur unicytokine (anti IL1 ou anti IL6 ou anti TNF).
4) rangées 7 et 8 : puits revêtus avec anticorps plurispécifiques (anti IL1 + anti IL6 + anti TNF) + standard unicytokines TNF (0, 100, 500, et 2000 pg/ml) ou échantillons (EC1 et EC2) + traceur uniticytokine (anti IL1 ou anti IL6 ou anti TNF).
5) rangées 9 et 10 : puits revêtus avec anticorps unispécifiques anti IL1 (ou anti IL6 ou anti TNF) + standard unicytokine (IL1 ou IL6 ou TNF) ou échantillons (EC1 et EC2) + traceur unicytokine (anti IL1 ou anti IL6 ou anti TNF).
6) rangées 11 et 12 : puits revêtus avec anticorps non spécifiques + échantillons (EC1 et EC2) + traceur unicytokine (anti IL1 ou anti IL6 ou anti TNF).

Chaque dosage d'intérêt est représenté par une feuille d'expérience représentant :
- le plan de l'expérience
- les densités optiques obtenues après 4 heures de culture
- les densités optiques obtenues après 24 heures de culture.

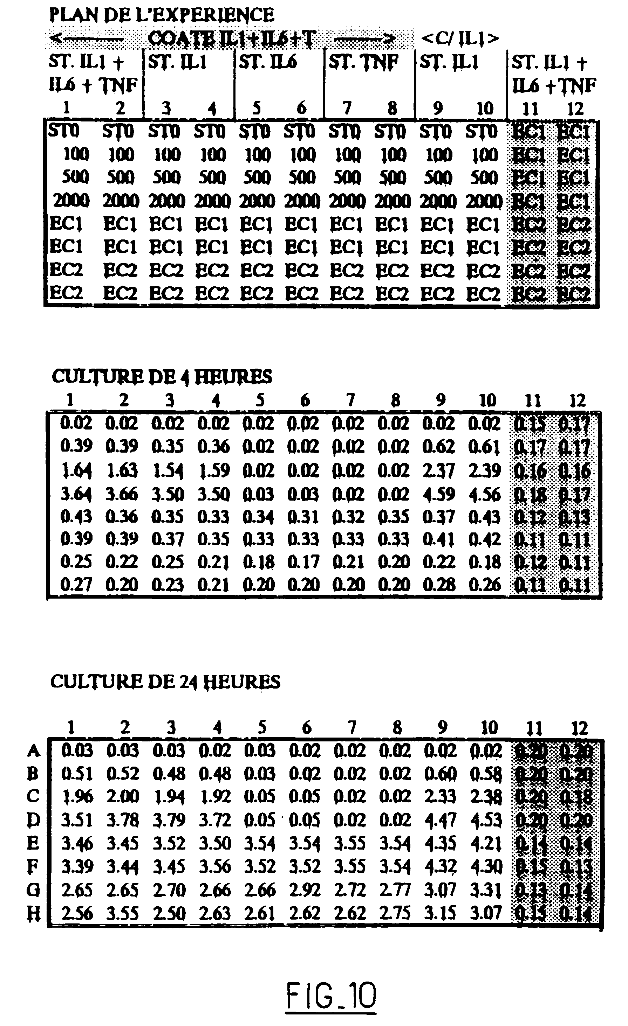

Les résultats sont présentés aux figures 10 à 12

### C) Interprétation des résultats :

1) L'allure des courbes standards obtenues suivant le format multicytokine-multitraceur est entièrement satisfaisante pour les trois cytokines mesurées : IL1, IL6 et TNFa.
2) Par rapport au format unicytokine, les signaux obtenus dans le format multicytokines-multitraceur sont légèrement amplifiés (cela se marque particulièrement pour l'IL6), ce qui sensibilise le dosage sans affecter la mesure car l'effet est observé aussi bien pour le standard que pour l'échantillon.
3) La spécificité du signal observé par rapport à la cytokine mesurée est complète. Ex. : lors du dosage d'ILl sur une plaque coatée avec des anticorps spécifiques de l'ILl en présence d'un multitraceur anti IL1 + anti IL6 + anti TNF, il n'y a pas de signal en présence de standards TNF ou IL6, mais seulement en présence de standard IL1 ou du multistandards IL1 + IL6 + TNF.
4) La production et le dosage in situ de TNF, de l'IL1 et de l'IL6 après 4 heures et 24 heures de culture dans le format multicytokine-multitraceur sont entièrement satisfaisants et comparables aux productions et aux dosages obtenus dans le format unicytokine.
5) Le bruit de fond observé avec le traceur multicytokine est toujours suffisamment bas (<0.2 unités de densité optique) et comparable à celui d'un traceur unicytokine.
6) La reproductibilité des résultats est excellente.

En conclusion, que ce soit selon le principe du traceur multiparamètres ou selon le principe du multi revêtement ("multicoating"), le format donne des résultats parfaitement cohérents et reproductibles.

### EXEMPLE 3 : STABILITE DES ELEMENTS DU DOSAGE DANS LES CONDITIONS DE CULTURE. ILLUSTRATION PAR LA CINETIQUE DE DOSAGE IN SITU DU TNFa

### Dosage in situ du TNFa suivant le format unicytokine (cf. exemple 1) pendant des temps de culture de 4, 24, 48 et 78 heures.

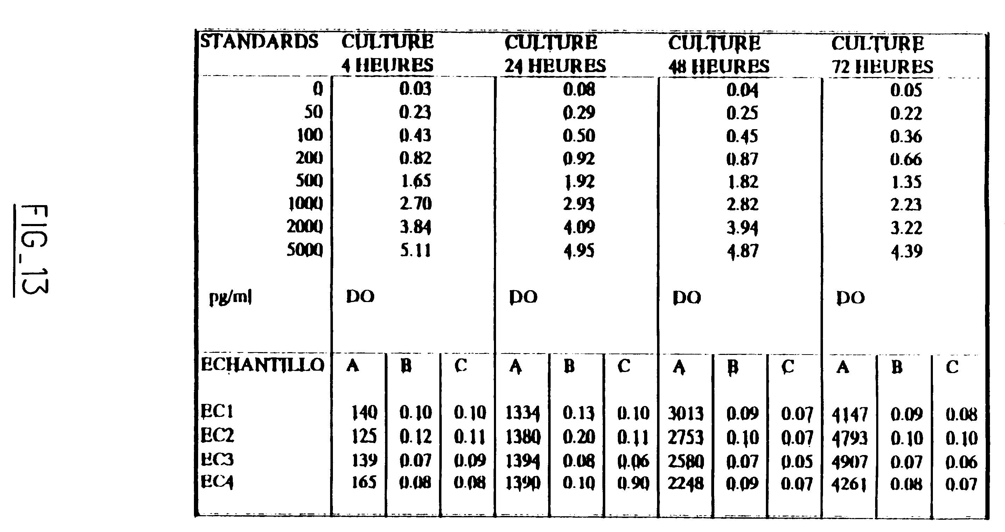

1) Explications de l'expérience :
   Des courbes standard TNFa(en double) et 4 échantillons (en quadruple) sont incubés suivant la procédure unicytokine pendant des temps de culture de 4 à 72 heures. Le contrôle des signaux non spécifiques est assuré par l'incubation des échantillons soit en absence d'anticorps traceur, soit dans des puits coatés avec des anticorps non spécifiques.
2) Plan de l'expérience :
   La feuille de résultats montre :
   - les courbes standard obtenues après 4, 24, 48 ou 72 heures de culture (en unités de densité optique)
   - les valeurs correspondantes de TNFₐ produit in situ pendant les temps de culture correspondants (en pg/ml).
   - les signaux non spécifiques observés en absence d'anticorps traceur ou d'anticorps coatés spécifiques en unités de densité optique).
3) Résultats : ils sont présentés figure 13

   a. Le profil des courbes standard reste très stable pendant toute la durée de la cinétique même après 72 heures de culture à 37 ° C, les DO correspondant aux différents points standards sont toujours supérieurs à 80 % des DO obtenues après 4 heures de culture.
   b. Les productions in situ de TNF croissent régulièrement en fonction de la durée de la culture.
   c. Les signaux non spécifiques sont toujours faibles (le plus souvent <0.1 unité de DO).

   En conclusion l'intégration des étapes de dosages aux conditions de culture n'est pas empêchée par des problèmes de stabilité dans le procédé selon l'invention, et ceci même pour une phase de production s'étendant à plus de 3 jours.

### EXEMPLE 4 : CONDITIONS DE LAVAGE

Les conditions de lavage sont importantes car elles doivent permettre d'éliminer complètement les cellules des puits de culture tout en préservant les complexes antigènes-anticorps. Dans l'expérience présentée,on a testé 8 conditions de lavage différentes en utilisant comme modèle le dosage in situ du TNFa suivant le format unicytokine.

Description de l'expérience :

Un échantillon de sang complet est réparti sur deux plaques de microtitration anti-TNF suivant le format unicytokine (cf. exemple 1).
Une plaque est incubée pendant 4 heures et l'autre pendant 24 heures.
Les cultures sont arrêtées après 4 ou 24 heures par lavage suivant les conditions décrites dans le tableau. L'efficacité du lavage est déterminée par les DO observées en absence d'anticorps traceurs ou en absence d'anticorps coatés spécifiques (DO minimales) sans altération du signal spécifique en présence d'anticorps traceurs et coatés spécifiques (DO maximales).

Plan de l'expérience :
- un tableau représentant les 8 conditions de lavage.
- un schéma de plaque localisant les puits suivant le lavage qu'ils ont subi.
- un schéma de plaque représentant les DO lues après 4 ou 24 heures de culture.

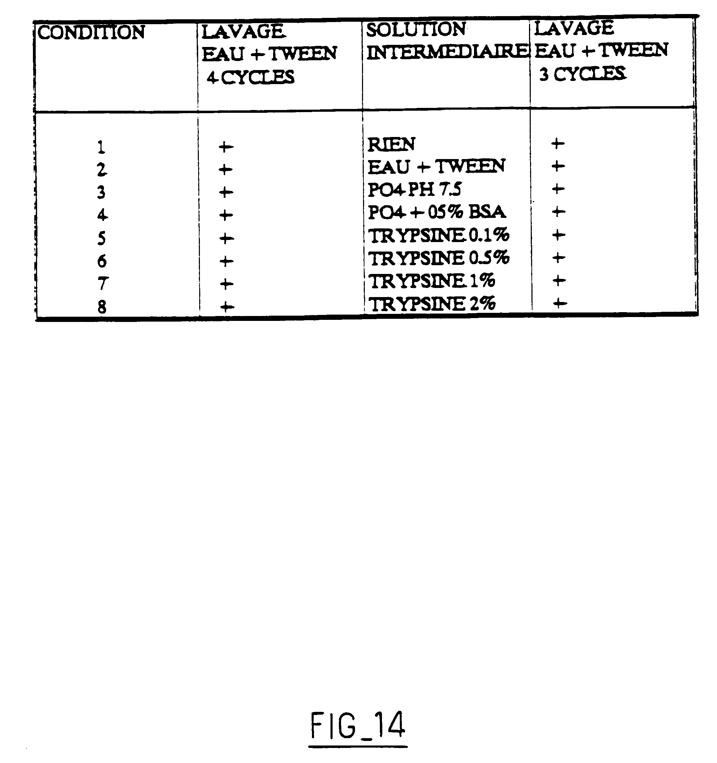

Résultats : ils sont présentés aux figures 14 à 16
1) De l'eau + un détergent comme le Tween 20 n'élimine pas totalement les cellules, ce qui entraîne un signal non spécifique (DO > 0.5). Un lavage intermédiaire de la même composition n'a pas d'effet.
2) Le tampon phosphate avec ou sans albumine bovine ajouté entre deux lavages à l'eau + Tween diminue le signal non spécifique de façon satisfaisante.
3) Le même résultat est obtenu avec les différentes solutions de trypsine sans altération du signal spécifique pour les concentrations que nous avons utilisées.

### EXEMPLE 5 : CORRESPONDANCE ENTRE RESULTATS PAR DOSAGE IN SITU ET DOSAGE CLASSIQUE.

Au cours d'une expérience, les productions in situ d'IFNg suivant le format unicytokine (cf. exemple 1)
et les productions par dosages classiques correspondantes, c'est-à-dire après culture dans un support séparé puis dosage dans une étape séparée, d'un groupe de donneurs normaux et d'un groupe de sujets pathologiques ont été comparées.

La population normale se composait de 6 échantillons et la population pathologique de 9 échantillons dont deux patients ayant subi une greffe de foie (GF), deux cancers du colon (CC) et cinq greffes de moelle (GM) à différents stades après l'intervention.

Les valeurs obtenues pour les différents échantillons après 4 ou 24 heures de culture sont représentées en tableaux et en graphique pour les deux types de cultures. Les échantillons s'écartant du rang des normaux sont marqués de * (valeurs < normale) ou de ** (valeurs > normale).

Les corrélations entre les deux types de cultures ont également été calculées.

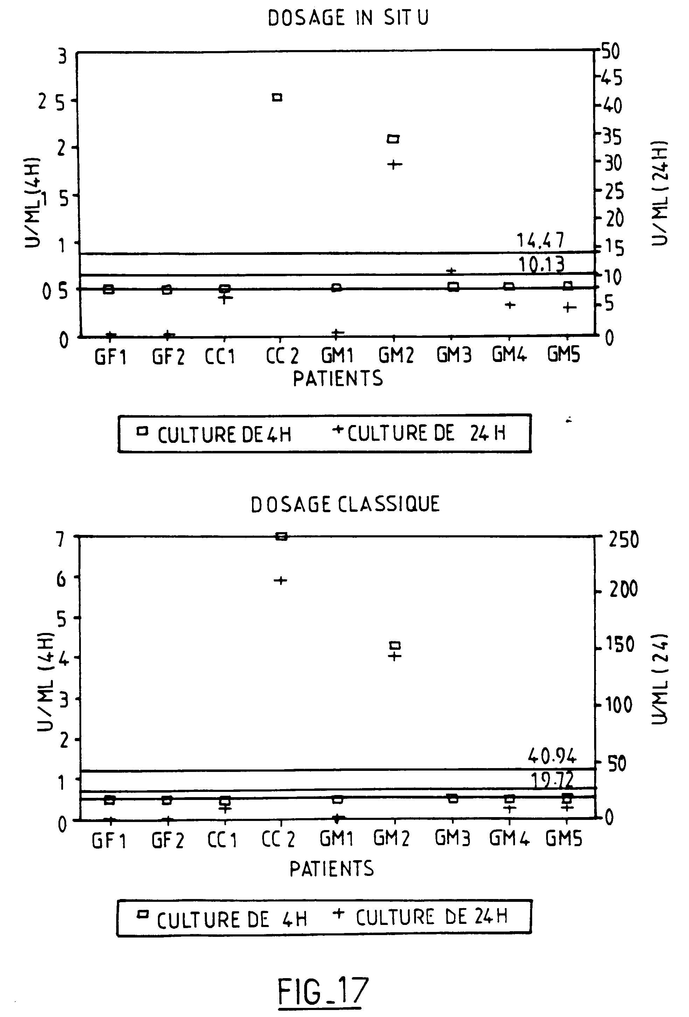

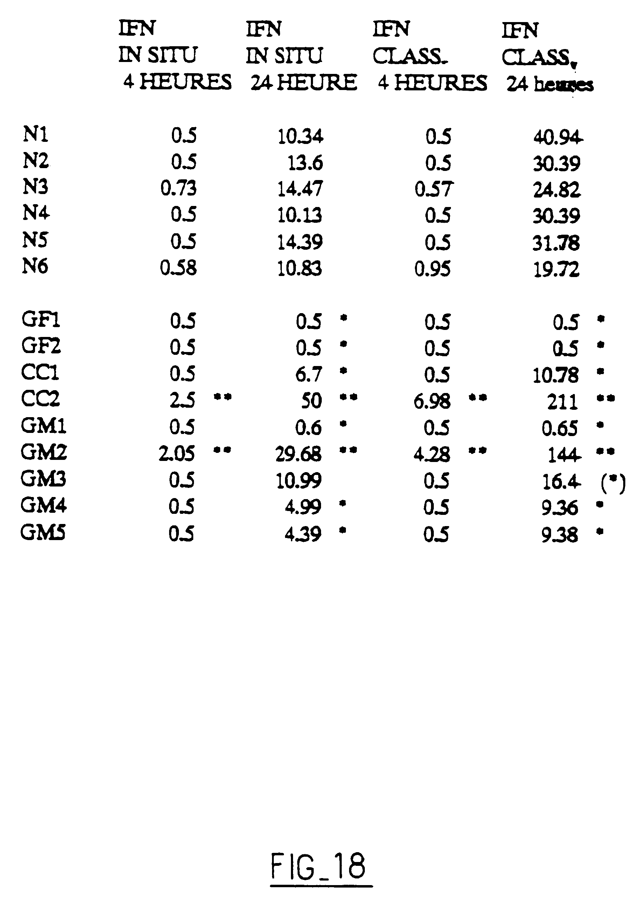

Résultats : ils sont présentés aux figures 17 et 18
1) Les valeurs d'IFNg obtenues par dosage classiques sont supérieures aux valeurs obtenues par dosage in situ.
2) Cependant la corrélation entre les deux méthodes est excellente (r = 0.97 avec p < 0.0001) et très significative.
3) Les pathologiques se distinguent de la même manière par les deux méthodes.
4) La méthode de dosage in situ est capable de distinguer les productions normales des productions anormales d'IFNg.

### EXEMPLE 6 : APPLICATION A LA POLYARTHRITE RHUMATOIDE.

La production de cytokines par le sang complet dilué de patients souffrant d'une polyarthrite rhumatoïde a été comparée à celles de témoins normaux ou de patients souffrant d'arthrose.

Il a été montré que les profils de production des cytokines ainsi mesurés sont significativement modifiés dans les états pathologiques sus mentionnés.

En particulier, les patients atteints de polyarthrite rhumatoïde présentent une production d'IL1, de TNF, d'IL6 et d'IL2 nettement accrue par rapport aux normaux, et significativement accrue par rapport aux patients arthrosiques. Ceux-ci ont des valeurs supérieures aux normaux, en particulier pour la production d'IL2 et, dans une moindre mesure, de TNF et d'IL6.

De manière intéressante, la production d'IFN Y est comparable chez les normaux et les arthrosiques, mais très significativement diminuée chez les polyarthritiques. La production de GM-CSF est égale dans les 3 groupes.

Ces données permettent d'envisager l'évaluation physiopathologique et le suivi thérapeutique des patients atteints de ces affections. Dans le cadre diagnostic, cette approche pourrait également permettre de préciser la susceptibilité de certains patients à risques à développer une polyarthrite. Dans ce cadre, l'activation de la production de cytokines par des antigènes spécifiques impliqués dans la maladie pourrait être entreprise (par exemple collagène de type 2, Heat Shock protein, antigènes bactériens, ...).

### EXEMPLE 7 : APPLICATION A LA SCLEROSE EN PLAQUE (SEP)

Les patients souffrant de sclérose en plaque présentent habituellement des périodes de latence de la maladie, suivies de phases de rechute et d'exacerbation. Ces phases ne sont actuellement pas prédictibles et sont reconnues au moment où les atteintes neurologiques cliniques sont évidentes, et souvent irréversibles.

Des patients souffrant de SEP ont été suivis prospectivement par la mesure de la production de cytokines par leur sang complet stimulé in vitro.

On a observé plusieurs semaines (habituellement 2 à 6 semaines, parfois jusqu'à 12 semaines) avant l'apparition de nouvelles lésions neurologiques (mesurées par le score de Kurtzke), une augmentation très significative de la production de TNF par le sang stimulé de ces patients.

Il est donc possible de mesurer l'activation du système immunitaire qui précède l'apparition des atteintes neurologiques cliniques, et donc d'instaurer précocement un traitement (par exemple corticoïdes) avant que les lésions neurologiques irréversibles soient apparues.

Un tel diagnostic précoce est susceptible d'améliorer considérablement le pronostic de ces patients.

### EXEMPLE 8 : ETUDE D'AGENTS PHARMACOLOGIQUES A L'AIDE DE LA METHODE

La production de cytokines par le sang complet stimulé de patients atteints d'arthrite rhumatoïde est mesurée en présence de concentrations croissantes de médicaments modulant l'activité de la maladie, l'allochrysine et le méthotrexate.

La production d'IL1 est diminuée d'une manière dose dépendante par l'allochrysine, et celle d'IFN Y par le méthotrexate.

On peut donc étudier in vitro l'activité d'agents pharmacologiques sur les cellules de patients pour déterminer l'activité de l'agent, la capacité de réponse des cellules du patient à différents agents et les doses actives.

### EXEMPLE 9 : COMBINAISON D'UN BIODOSAGE AU DOSAGE IN SITU SELON LE FORMAT DE L'INVENTION

Différentes molécules peuvent être mesurées par une quantification de leur activité biologique exercée sur une cellule cible. L'activité mesurée peut être une modification physiologique de la cellule cible (mort de la cellule -par exemple dans le cas du bioassay TNF/L929- ou prolifération de la cellule -par exemple dans le cas du bioassay pour l'IL2 basé sur la prolifération de cellules T cible qui est mesurée indirectement par l'incorporation de thymidine H3-) ou la production d'une protéine spécifique par la cellule cible (par exemple, mesure de l'activité biologique de l'IL4 par la quantification du CD23 membranaire ou éventuellement de sa forme soluble exprimée par une lignée lymphocytaire B cible).

Dans la plupart des cas, les bioassay sont réalisés suivant des procédures multiétapes longues et fastidieuses, de sorte que les dosages immunologiques sont de plus en plus préférés aux bioassay. Cependant, dans certains cas, par exemple lorsque des formes biologiquement actives ou inactives d'une même molécule peuvent coexister dans le même échantillon, la mesure de l'activité biologique peut apporter une information complémentaire au dosage immunologique de la molécule.

Le format technologique basé sur le principe du dosage in situ est particulièrement bien adapté pour réaliser des dosages biologiques combinant l'activation biologique d'une lignée cellulaire cible par la molécule d'intérêt et le dosage de la molécule induite, produite par la cellule cible, en une procédure simplifiée uniétape.

### EXEMPLE 9-1 : MESURE DE L'IL4 BIOACTIVE

Les échantillons contenant l'IL4 bioactive à mesurer sont incubés dans des puits coatés avec les anticorps anti-CD23 en présence des anticorps anti-CD23 traceurs participant au dosage du CD23 soluble et en présence d'un nombre déterminé de cellules de la lignée Ramos. En présence d'IL4 bioactive, les cellules Ramos produisent du CD23 qui est mesuré suivant le principe du dosage in situ. Dans ce cas, la quantité de CD23 produite est directement proportionnelle à la quantité d'IL4 présente dans l'échantillon.

### EXEMPLE 9-2 : CARACTERISATION DANS LE SERUM DE PATIENTS CANCEREUX, DE MOLECULES INDUISANT LA PRODUCTION DE CYTOKINES PAR DES LIGNEES CELLULAIRES.

Le sérum des patients est incubé dans des puits coatés avec les anticorps anti-cytokines et en présence des anticorps traceurs participant au dosage de la ou des cytokines d'intérêt et en présence d'un nombre déterminé de cellules d'une lignée choisie. Les cytokines éventuellement produites sont alors mesurées suivant le principe du dosage in situ, leur production étant le reflet de la présence dans le sérum de ces patients, de la molécule associée à un état cancéreux.

### EXEMPLE 10 : APPLICATION A UN TEST D'IMMUNOCOMPATIBILITE

Les tests d'immunocompatibilités sont réalisés avant greffes entre les cellules mononuclées (PBMC) du donneur et du receveur. En pratique, les PBMC du donneur et du receveur sont isolées sur ficoll. Les PBMC du receveur sont cultivées en présence des PBMC irradiées du donneur en présence de thymidine H3. Lorsque les cellules sont incompatibles (antigènes d'histocompatibilités différents) les PBMC irradiées du donneur activent les PBMC du receveur avec comme conséquence une prolifération de ces cellules qui est mesurée par l'incorporation de thymidine H3 après plusieurs jours de culture.

La procédure de ces tests est lourde, compliquée et prend beaucoup de temps (4 à 5 jours).

Or, les PBMC activées du receveur peuvent produire des cytokines qui reflètent cette activation et conséquemment l'incompatibilité cellulaire.

On réalise un test d'immunocompatibilité basé sur le principe du dosage in situ de cytokines produites par les PBMC du receveur en présence des cellules du donneur potentiel.

Dans ce cas, les PBMC (éventuellement le sang complet) du receveur sont incubées en présence des PBMC irradiées du donneur dans des puits coatés avec les anticorps anti-cytokines et en présence des anticorps traceur anti-cytokines participant au dosage de la ou des cytokines d'intérêt. La production de cytokines est mesurée suivant le principe du dosage in situ. La production de cytokine mesurée est une mesure indirecte de l'incompatibilité des cellules du donneur et du receveur.

### EXEMPLE 11 : TEST DE DETECTION VIRALE

Même chez un individu infecté, les particules virales circulantes sont le plus souvent en quantité trop faible pour assurer leur détection directe par immunodosage dans le sérum. Une solution possible est d'incuber le sérum en présence des cellules réceptives au virus. L'infection de ces cellules se traduit par une production abondante de particules virales qui peut être secondairement mesurée dans le surgageant (ou évaluée par la toxicité cellulaire). La réalisation de ce test dans le format selon l'invention permet de réaliser la culture et le dosage sur le même support et éviter des manipulations supplémentaires avec les risques de contamination qui y sont liées.

Il est également possible de mettre en culture des cellules du sang d'un patient contaminé (par exemple par un virus herpès, un rétrovirus, etc...) et d'activer ces cellules par des mitogènes pour amplifier la production virale in vitro. Cette production peut alors être directement mesurée dans un format selon l'invention (la culture se faisant en présence d'un support aux anticorps de capture et d'un traceur adéquat). Le test pourrait être sensibilisé par l'addition, par exemple, d'anticorps neutralisants anti-interférons dans le milieu de culture.

### EXEMPLE 12 : DESENSIBILISATION

### Illustration par la désensibilisation du dosage in situ de l'IFNg

### Dosage in situ de l'IFNg suivant le format unicytokine (c.f. exemple 1) désensibilisé par des doses croissantes d'anticorps traceur froid.

Principe de l'expérience:
4 courbes standard ont été réalisées en présence de concentrations croissantes d'anticorps traceur froid (0; 10; 50; et 250 nanogrammes/puits) et incubées et dosées in situ suivant le format unicytokine décrit précédemment pendant 4 et 24 heures de culture. Parallèlement aux courbes standards, deux échantillons de sang complet enrichis par des doses croissantes d'IFNg exogène correspondant aux mêmes concentrations que dans les standards (de 0 à 1000 u/ml) ont été incubés et dosés de la même façon en présence des mêmes concentrations d'anticorps traceur froid.

Les courbes standard désensibilisées (incubées en présence de 10; 50 ou 250 ng/ml d'anticorps traceur froid) ont été lues sur la courbe standard de référence non désensibilisée (sans anticorps traceur froid). De même, les courbes standard en sang complet ont également été lues sur cette même courbe standard de référence.

Les droites de régression linéaires entre les différentes courbes et la courbe de référence ont été calculées afin de déterminer les facteurs de correction (= au coefficient de X dans l'équation de la droite ou à l'inverse de la pente) associés aux différentes désensibilisations. Les facteurs obtenus à partir des courbes en sang complet ont été comparés aux facteurs correspondants obtenus à partir des courbes standard afin d'établir la validité de ces facteurs de correction dans le sang complet activé.

Plan de l'expérience :
- Les courbes standard obtenues en présence de concentrations croissantes d'anticorps froid sont représentées dans le tableau n° 1.
- Les coefficients de X des différentes droites de régressions calculées (équivalant aux facteurs de corrections associés aux différentes désensibilisations) sont représentés dans le tableau n° 2.

**TABLEAU N° 1 :**

| COURBES STANDARDS | | | | |
|---|---|---|---|---|
| Incubation 4 heures | | | | |
| | Anticorps froid (ng/puits) | | | |
| | 0 | 10 | 50 | 250 |
| Std 0 | 0.028 | 0.028 | 0.025 | 0.027 |
| Std 0.5 | 0.112 | 0.076 | 0.042 | 0.031 |
| Std 1 | 0.201 | 0.094 | 0.055 | 0.032 |
| Std 2 | 0.423 | 0.222 | 0.097 | 0.039 |
| Std 5 | 1.019 | 0.528 | 0.218 | 0.071 |
| Std 10 | 1.974 | 0.972 | 0.428 | 0.118 |
| Std 20 | 3.391 | 1.851 | 0.773 | 0.216 |
| Std 50 | 4.805 | 3.464 | 1.733 | 0.492 |
| Std 100 | 5.154 | 4.805 | 2.901 | 0.945 |
| Std 200 | 5.251 | 5.419 | 4.173 | 1.622 |
| Std 500 | 5.335 | 5.521 | 5.274 | 3.009 |
| Std 1000 | 5.365 | 5.455 | 5.449 | 4.089 |

| Incubation 24 heures | | | | |
|---|---|---|---|---|
| | Anticorps froid (ng/puits) | | | |
| | 0 | 10 | 50 | 250 |
| Std 0 | 0.033 | 0.031 | 0.027 | 0.032 |
| Std 0.5 | 0.142 | 0.104 | 0.055 | 0.035 |
| Std 1 | 0.223 | 0.168 | 0.073 | 0.038 |
| Std 2 | 0.471 | 0.312 | 0.146 | 0.055 |
| Std 5 | 1.172 | 0.722 | 0.319 | 0.093 |
| Std 10 | 2.188 | 1.275 | 0.575 | 0.162 |
| Std 20 | 3.588 | 2.214 | 1.066 | 0.348 |
| Std 50 | 5.091 | 3.913 | 2.021 | 0.688 |
| Std 100 | 5.452 | 5.037 | 3.173 | 1.259 |
| Std 200 | 5.597 | 5.573 | 4.478 | 2.021 |
| Std 500 | 5.627 | 5.663 | 5.543 | 3.377 |
| Std 1000 | 5.663 | 5.669 | 5.633 | 4.328 |

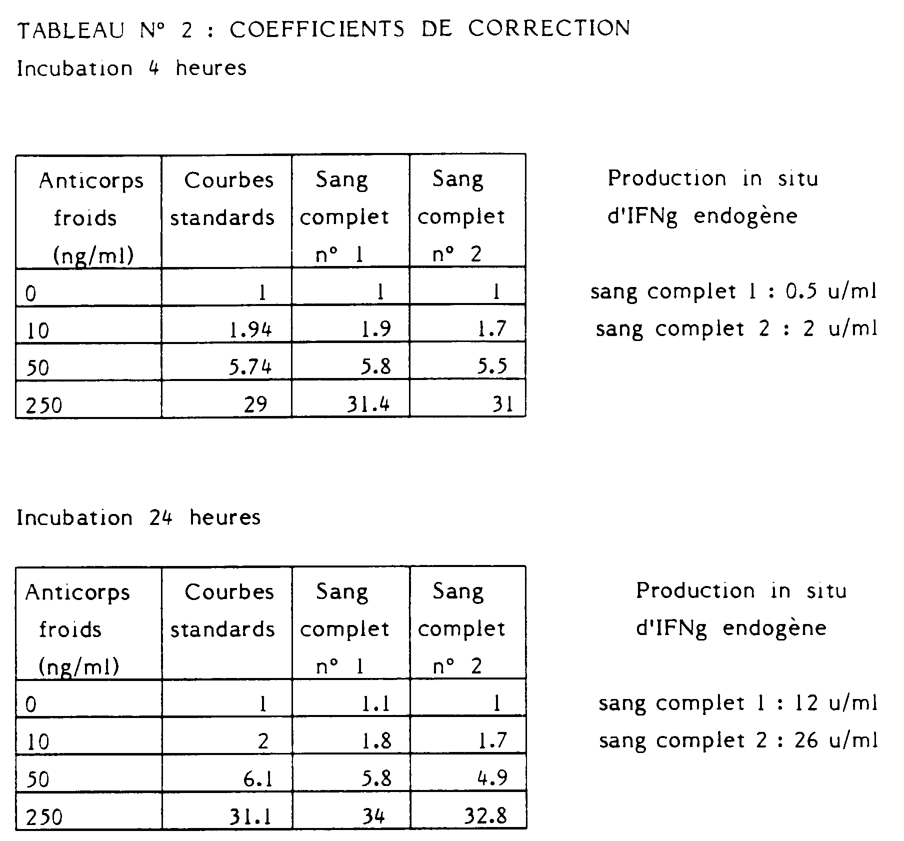

### Remarque:

Les coefficients de correction correspondent aux A des équations AX+B des droites de régression entre les courbes standards désensibilisées ou les courbes en sang complet et la courbe référence non désensibilisée.

### Résultats:

1) Le tableau montre que le signal (densité optique) diminue proportionnellement à la quantité d'anticorps froid présente dans le traceur et cela pour les incubations de 4 heures comme pour celles de 24 heures. La courbe non désensibilisée est fonctionnelle entre 0.5 et 50 u/ml, ensuite elle plafonne. La courbe désensibilisée à 10 ng est fonctionnelle entre 1 et 100 u/ml, ensuite elle plafonne. La courbe désensibilisée à 50 ng est fonctionnelle entre 2 et 500 u/ml, ensuite elle plafonne. La courbe désensibilisée à 250 ng est fonctionnelle entre 10 et 1000 u/ml.
2) Les coefficients de correction entre les courbes standards désensibilisées et la courbe référence non désensibilisée sont proches de 2 pour 10 ng/puits d'anticorps froid, de 6 pour 50 ng/puits d'anticorps froid et de 30 pour 250 ng/puits d'anticorps froid.
3) Les coefficients calculés à partir des courbes en sang complet sont fort semblables aux coefficients correspondants calculés à partir des courbes standard désensibilisées.
4) En conclusion, les résultats de ces expériences montrent qu'il est possible de mesurer in situ la production de quantités élevées d'IFNg en pratiquant le dosage en présence d'une quantité définie d'anticorps froid. Dans ce cas, la valeur extrapolée par rapport à une courbe standard non désensibililsée doit être multipliée par un facteur de correction qui dépend de la quantité d'anticorps froid utilisée dans le dosage. Dans l'exemple présenté, nous voyons qu'en dosant un échantillon en présence de 250 ng/puits d'anticorps froid, il est possible de mesurer des productions de 1500 u/ml d'IFNg en utilisant une courbe standard qui n'est fonctionnelle que jusqu'à 50 u/ml.

### EXEMPLE 13 : FORMAT PARTICULIER DE DOSAGE UNICYTOKINE

### 1. Matériel fourni sous forme de kit de dosage in situ unicytokine (format particulier)

- une plaque de microtitration revêtue avec des anticorps spécifiques de la cytokine que l'on désire mesurer et comprenant des barettes de micropuits fermées par des barrettes ("strips") de bouchons et contenant
   soit
   des standards séchés (par évaporation ou lyophilisation) précalibrés = "puits standards",
   soit
   des activateurs polyclonaux (LPS + PHA) séchés (par évaporation ou lyophilisation) en quantité déterminée (pour avoir 5 ug de PHA et 25 ug de LPS après reconstitution ) = "puits échantillons",
- des barrettes de micropuits fermées par des barrettes de bouchons, revêtues avec des anticorps non spécifiques de la cytokine que l'on désire mesurer et comprenant des activateurs polyclonaux (LPS + PHA) séchés (par évaporation ou lyophilisation) en quantité déterminée (pour avoir 5 ug de PHA et 25 ug de LPS après reconstitution) = "puits contrôles";
- une fiole contenant une solution 10 fois concentrée d'anticorps traceur spécifique de la cytokine à mesurer;
- une solution de milieu de culture (par exemple RPMI 1640);
- une solution de lavage A usuelle pour éliminer les complexes Ag-Ac n'ayant pas réagi;
- une solution de lavage B pour le détachement cellulaire;
- une fiole de solution concentrée de substrat de l'enzyme avec lequel l'anticorps traceur est marqué (par exemple : la peroxydase);
- une fiole de solution tampon pour diluer le substrat;
- une fiole d'H2SO4 pour bloquer la réaction enzymatique.

### 2. Procédure

On dilue l'anticorps traceur à 1/10 dans le volume requis de milieu de culture pour réaliser le test.

On ajoute 25 ul d'eau distillée aux puits standards et 25 ul de sang total aux puits échantillons et contrôles.
On ajoute 200 ul de la solution traceur en milieu de culture dans tous les puits.
On bouche les puits avec les bouchons.
On incube pendant X heures à 37°C.
On retire les bouchons.
On aspire le contenu de chaque puits et on lave 3 fois avec la solution de lavage A.
On ajouter 100 ul de solution de lavage B dans tous les puits.
On aspire le contenu de chaque puits et on lave 3 fois avec la solution de lavage A.
On ajoute 200 ul de solution de substrat (préparée justse avant l'emploi en diluant le substrat concentré dans le tampon substrat) à tous les puits.
On incube 15 minutes à température ambiante sous agitation (700 rpm).
On ajoute 50 ul d'H2SO4 à tous les puits.
On lit les barrettes sur un spectrophotomètre à 450 nm et 630 nm.

### 3. Expériences

- dosage des cytokines TNFalpha, IL6 et IFNgamma par la méthode décrite suivant le format particulier unicytokine.
   Pour chaque cytokine, une courbe standard est établie, ainsi que les densités optiques sur les puits éfhantillons et contrôles (en quadruple) après 4 heures (TNF et IL6) ou 24 heures (IFN) de culture.
   Pour chaque cytokine, une ciinétique de production en fonction du temps est effectuée. Les courbes représentent les moyennes de 4 sujets entourées des valeurs maximales et minimales obtenues.
   Détection de productions anormpales de cytokines chez des patients : Pour chaque cytokine, les valeurs normales de production ont été établies sur une population de 10 donneurs sains. Les valeurs extrêmes de la population normale sont comparées aux valeurs individuelles de 12 patients.

### 4. Conclusions

- Les courbes standards couvrent un large éventail de concentrations de cytokines (de 100 à 10 000 pg/ml pour le TNF, 200 à 20 000 pg/ml pour l'IL6, et 5 à 500 u/ml pour l'IFN). Dans ces conditions, les courbes obtenues sont entièrement satisfaisantes en termes de reproductibilité et de sensibilité.
- La production des cytokines présente une très bonne reproductibilité (qualité des quadruplets) ainsi qu'un signal bien démarqué par rapport aux contrôles qui sont toujours très bas.
- Des productions de cytokines entrant dans le rang des courbes standards peuvent être mesurées après 2, 4, ou 24 heures de culture pour le TNF, 4 ou 24 heures de culture pour l'IL6, et 24 ou 48 heures de culture pour l'IFN.
- Le système permet la détection de productions anormales de cytokines dans le sang complet de patients.

### LEGENDES RELATIVES AUX FIGURES :

### Légendes relatives à la Figure 1 :

### Exemple 1 : Format Unicytkine-IL1

Plan de l'expérience
Culture de 4 heures
Culture de 24 heures
Puits coatés avec anticorps anti-IL1 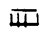
Puits coatés avec anticorps non spécifiques 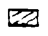
Interleukin I - Production in situ

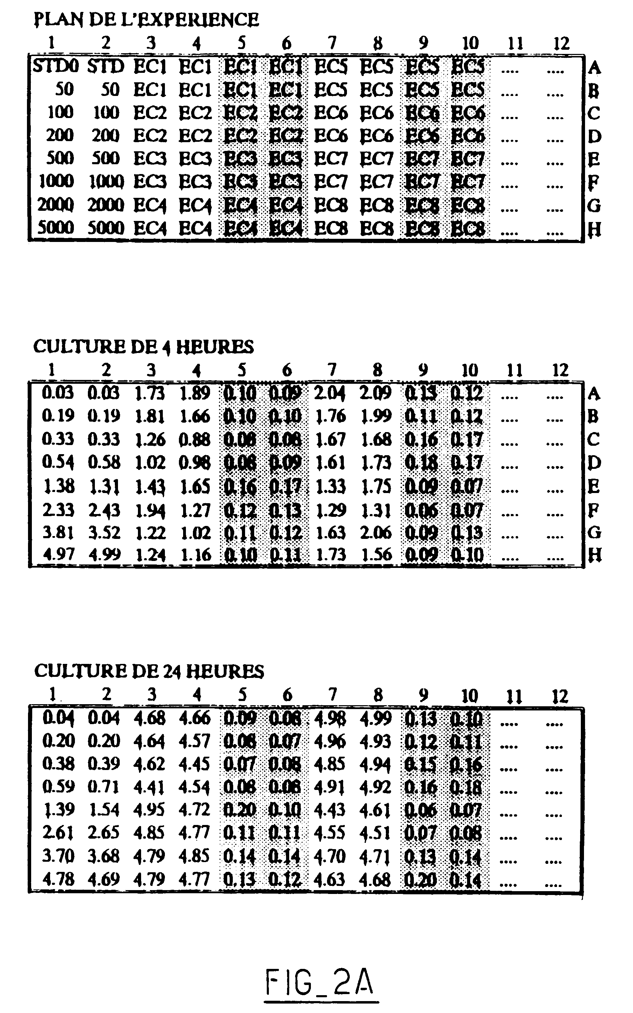

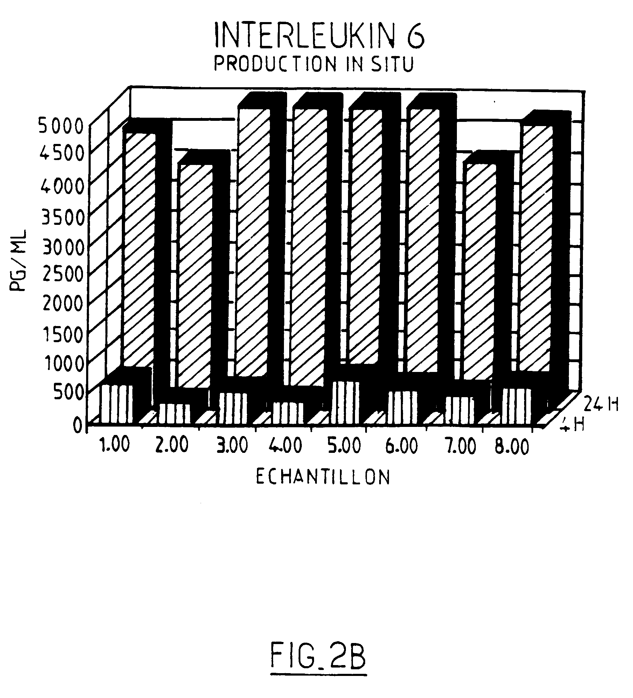

### Légendes relatives à la Figure 2 :

### Exemple 1 : Format Unicytokine-IL6

Plan de l'expérience
Culture de 4 heures
Culture de 24 heures
Puits coatés avec anticorps anti-IL6 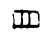
Puits coatés avec anticorps non spécifiques 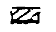
Interleukin 6 - Production in situ

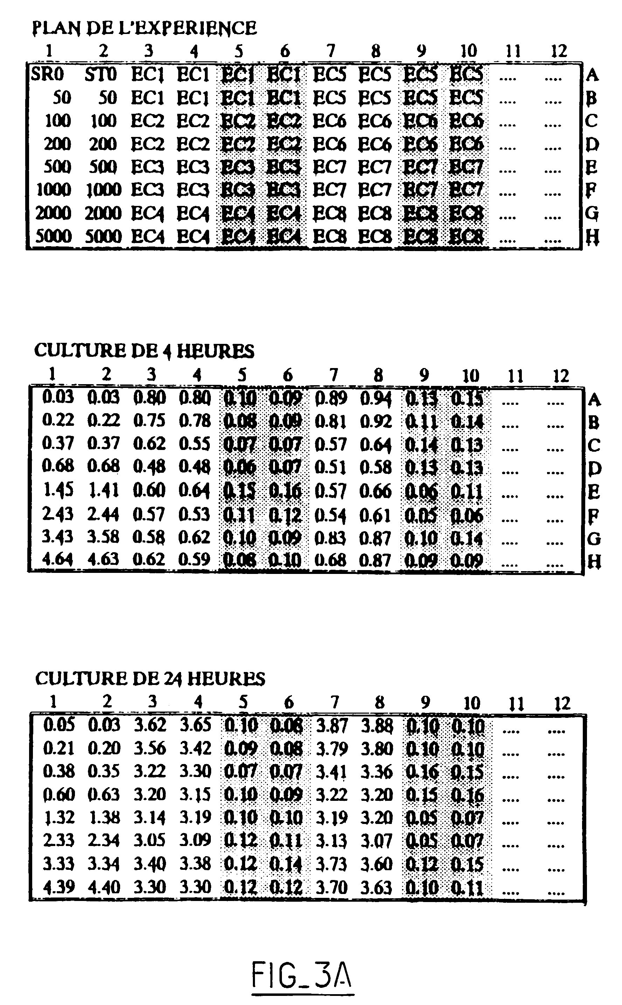

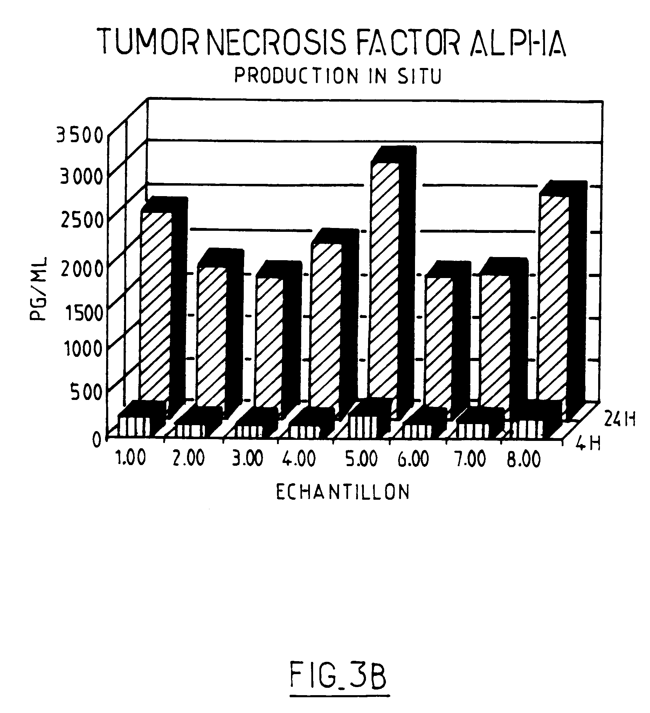

### Légendes relatives à la Figure 3 :

### Exemple 1 : Format Unicytokine-TNFa

Plan de l'expérience
Culture de 4 heures
Culture de 24 heures
Puits coatés avec anticorps anti-TNFa 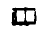
Puits coatés avec anticorps non spécifiques 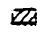
Tumor necrosis factor alpha - Production in situ

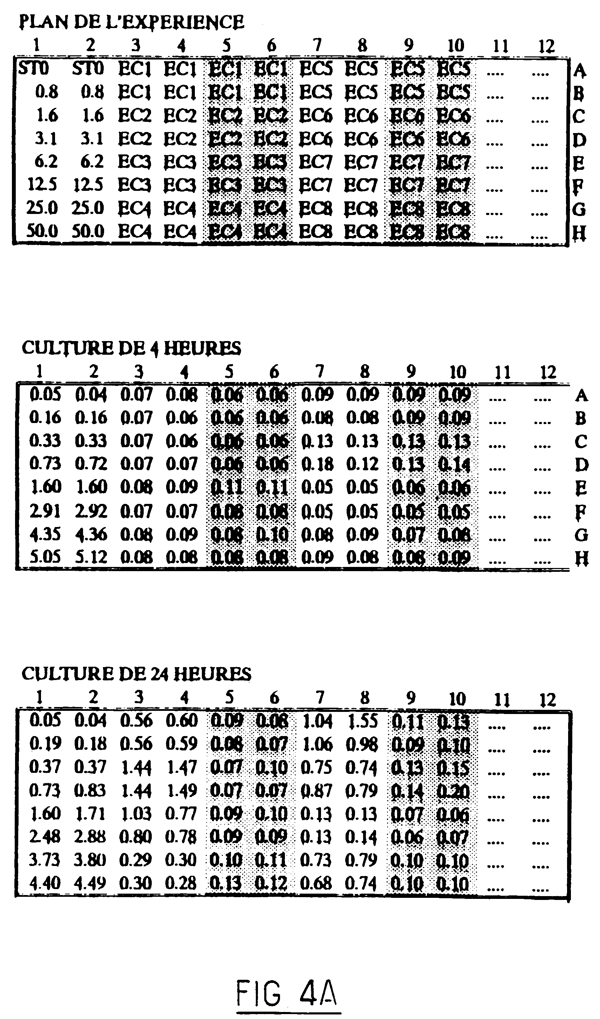

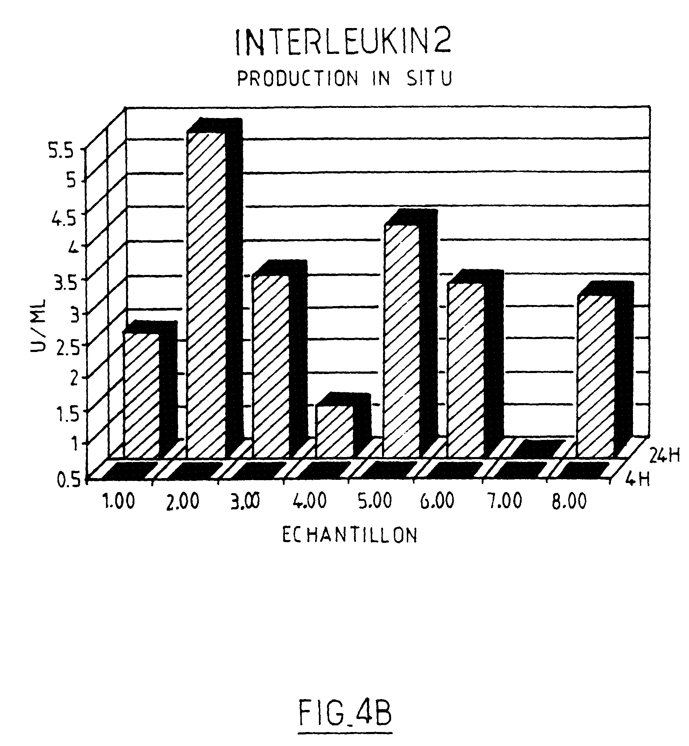

### Légendes relatives à la Figure 4 :

### Exemple 1 : Format unicytokine-IL2

Plan de l'expérience
Culture de 4 heures
Culture de 24 heures
Puits coatés avec anticorps anti-IL2 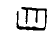
Puits coatés avec anticorps non spécifiques 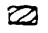
Interleukin 2 - Production in situ

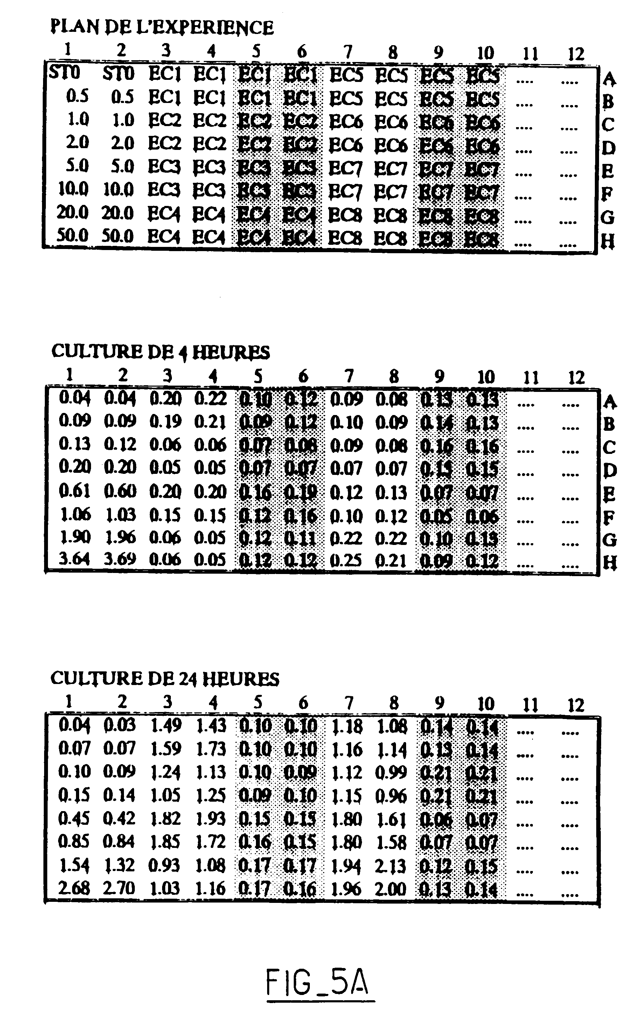

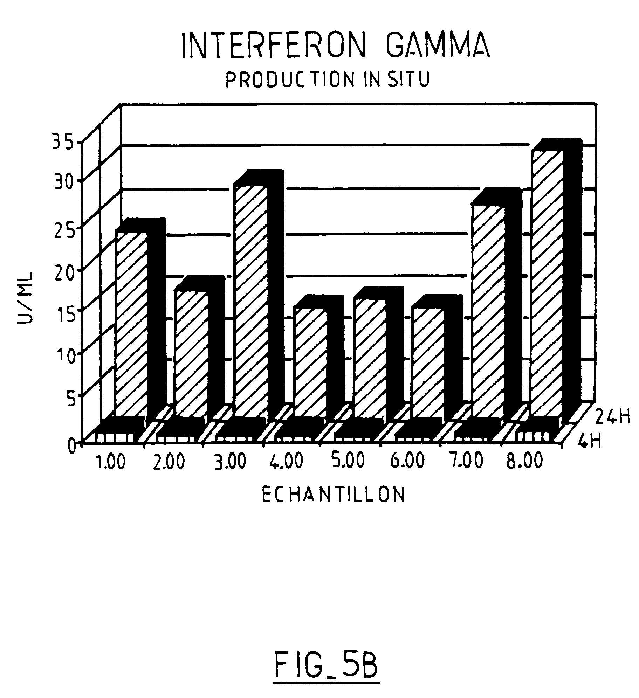

### Légendes relatives à la Figure 5 :

### Exemple 1 : Format unicytokine-IFNg

Plan de l'expérience
Culture de 4 heures
Culture de 24 heures
Puits coatés avec anticorps anti-IFNg 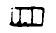
Puits coatés avec anticorps non spécifiques 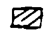
Interféron gamma - Production in situ

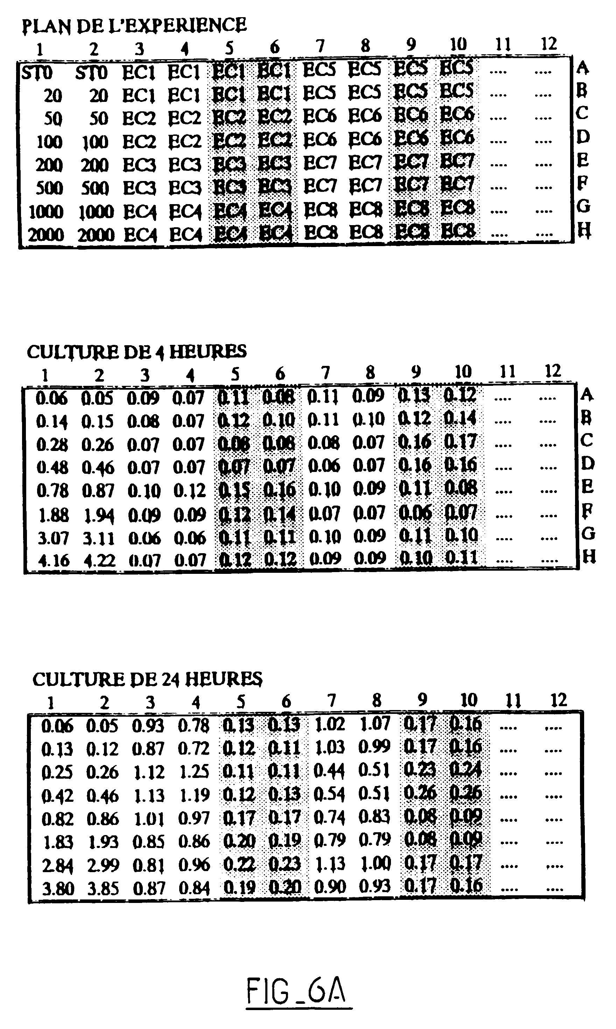

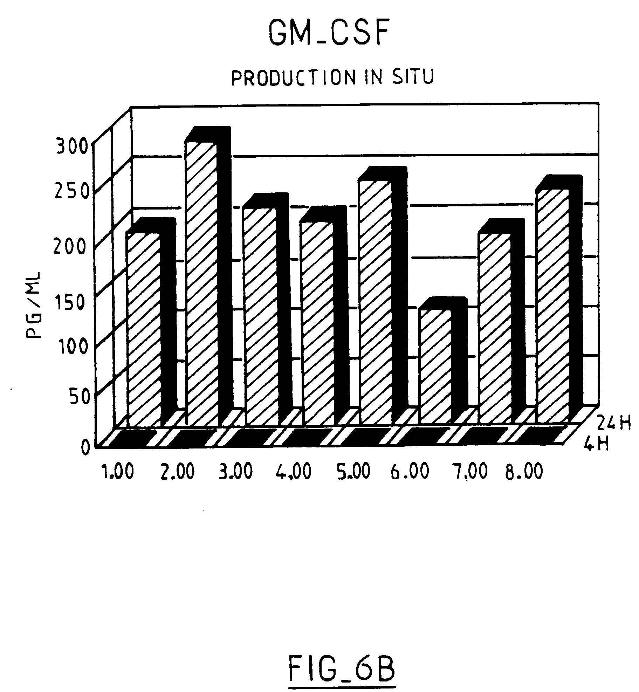

### Légendes relatives à la Figure 6 :

### Exemple 1 : Format unicytokine-GMCSF

Plan de l'expérience
Culture de 4 heures
Culture de 24 heures
Puits coatés avec anticorps anti-GMCSF 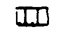
Puits coatés avec anticorps non spécifiques 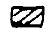
GM-CSF - Production in situ

### Légendes relatives à la Figure 7 :

### Exemple 2 : 2-1 Format multicytokine-multitraceur (IL1+IL6-TNF) A/Dosage de l'IL1

Plan de l'expérience
Culture de 4 heures
Culture de 24 heures
Puits coatés avec anticorps anti-IL1 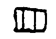
Puits coatés avec anticorps non spécifiques 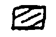

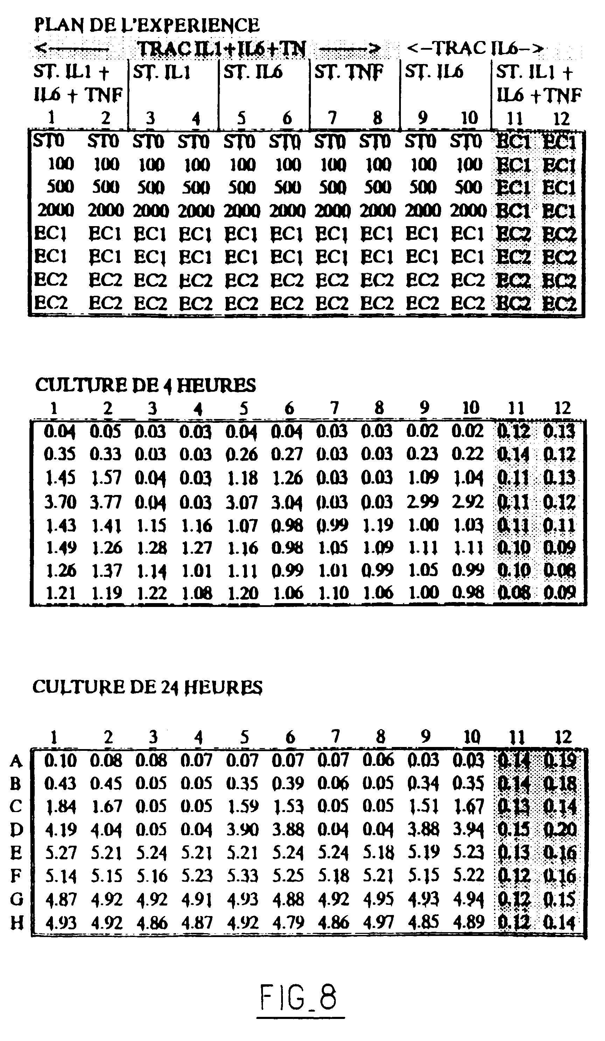

### Légendes relatives à la Figure 8 :

### Exemple 2 : 2-1 Format multicytokine-multitraceur (IL1+IL6+TNF) B/Dosage de l'IL6

Plan de l'expérience
Culture de 4 heures
Culture de 24 heures
Puits coatés avec anticorps anti-IL1 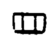
Puits coatés avec anticorps non spécifiques 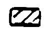

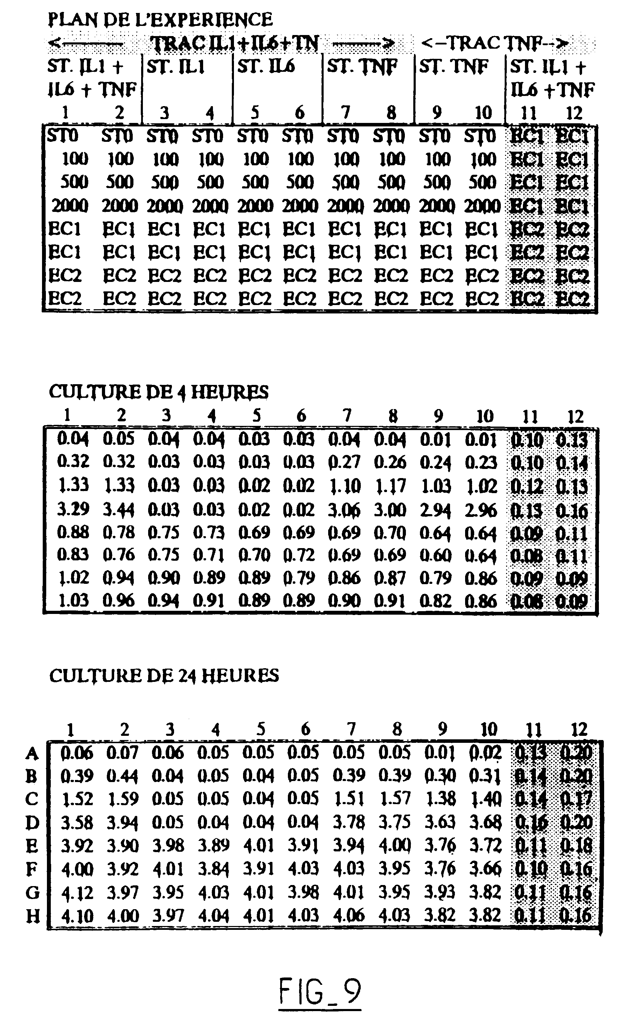

### Légendes relatives à la Figure 9 :

### Exemple 2 : 2-1 Format multicytokine-multitraceur (IL1+IL6+TNF) C/Dosage du TNF

Plan de l'expérience
Culture de 4 heures
Culture de 24 heures
Puits coatés avec anticorps anti-IL1 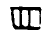
Puits coatés avec anticorps non spécifiques 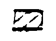

### Légendes relatives à la Figure 10

### Exemple 2 : 2-2 Format multicytokine-multicoatage (IL1-IL6-TNF) A/Dosage de l'IL1

Plan de l'expérience
Culture de 4 heures
Culture de 24 heures
Puits coatés avec anticorps non spécifiques 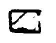

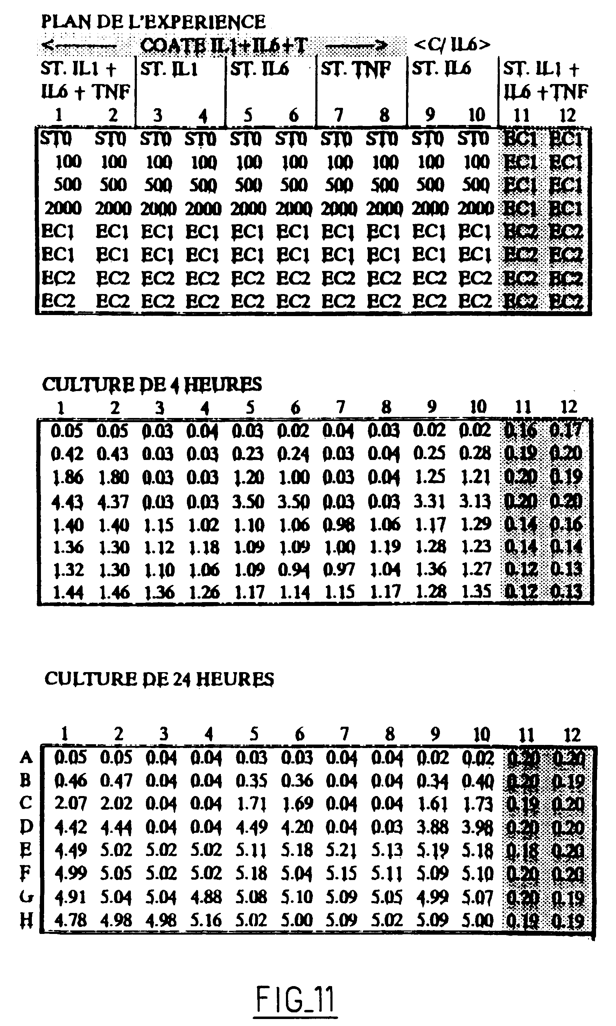

### Légendes relatives à la Figure 11 :

### Exemple 2 : 2-2 Format multicytokine-multicoatage (IL1+IL6-TNF) B/Dosage de l'IL6

Plan de l'expérience
Culture de 4 heures
Culture de 24 heures
Puits coatés avec anticorps non spécifiques 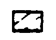

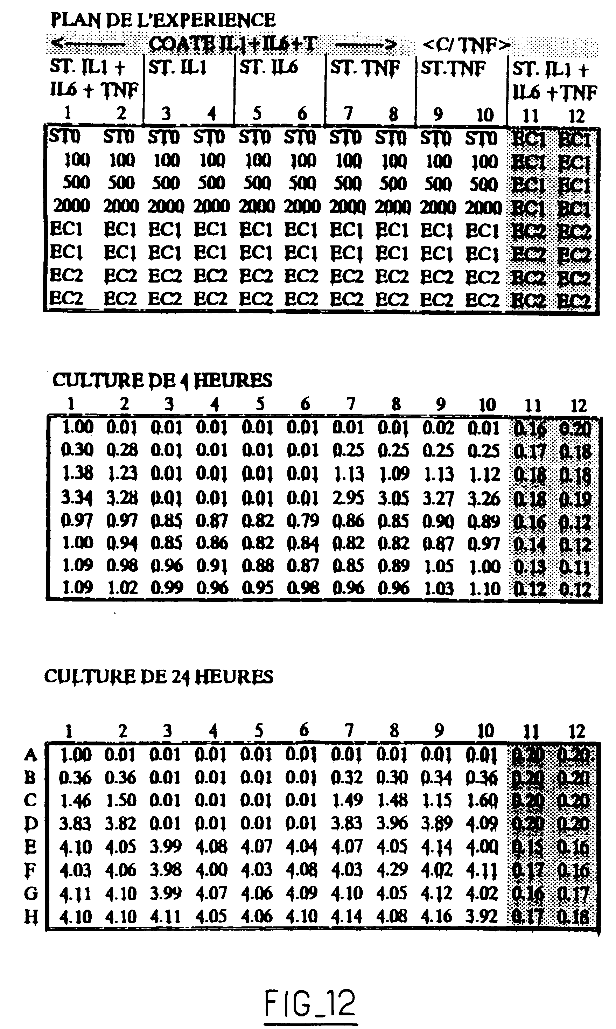

### Légendes relatives à la Figure 12 :

### Exemple 2 : 2-2 Format multicytokine-multicoatage (IL1+IL6+TNF) C/Dosage du TNF

Plan de l'expérience
Culture de 4 heures
Culture de 24 heures
Puits coatés avec anticorps non spécifiques 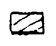

### Légendes relatives à la Figure 13 :

### Exemple 3 : Stabilité/Cinétique

- A =: Production in situ (en pg/ml)
- B =: Signal non spécifique ne absence d'anticorps traceur (en DO)
- C =: Signal non spécifique en absence d'anticorps coatés spécifiques (en DO)

### Légendes relatives à la Figure 14 :

### Exemple 4 : Conditions de lavage

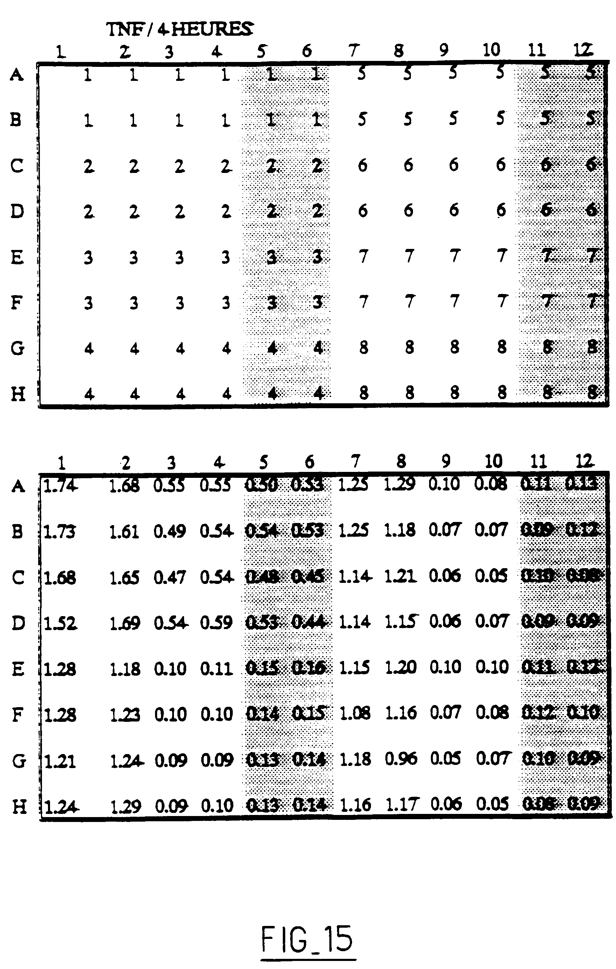

### Légendes relatives à la Figure 15 :

### Exemple 4 : Conditions de lavage

TNF/4 heures
Colonnes 1-4 et 7-10 = Puits coatés/anti-TNF ▭
Colonnes 5-6 et 11-12 = Puits coatés AC. non spécifique 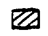
Anticorps traceur partout sauf dans les puits des colonnes 3-4 et 9-10

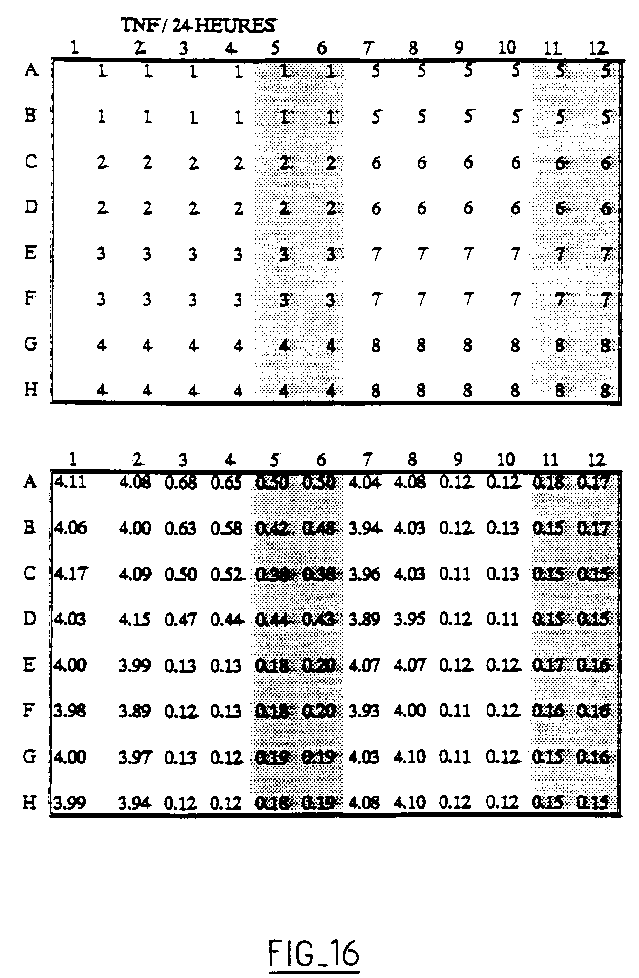

### Légendes relatives à la Figure 16 :

### Exemple 4 : Conditions de lavage

TNF/24 heures
Colonnes 1-4 et 7-10 = Puits coatés/anti-TNF ▭
Colonnes 5-6 et 11-12 = Puits coatés AC. non spécifique 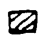
Anticorps traceur partout sauf dans les puits des colonnes 3-4 et 9-10

### Légendes relatives à la Figure 17 :

### Exemple 5 : Correspondance dosage in situ/ dosage classique

Interféron gamma - Dosage in situ
= culture de 4 H
+ culture de 24 H

Interféron gamma - Dosage classique
= culture de 4 H
+ culture de 24 H

### Légendes relatives à la Figure 18 :

### Interféron gamma - Dosage in situ versus dosage classique

N1 à N6 = 6 normaux
GF1 et GF2 = deux greffes du foie
CC1 = un cancer du côlon post-opératoire
CC2 = un cancer du côlon pré-opératoire
GM1 à GM5 = 5 greffes de moelle
REM : * = valeurs inférieures au rang des normaux
REM : ** = valeurs supérieures au rang des normaux

## Revendications

1. Procédé d'évaluation de la production par des cellules vivantes de molécule(s) telle(s) que des médiateurs solubles ou des éléments de particules infectieuses, dans lequel on effectue une culture cellulaire, et éventuellement une stimulation, pour induire ladite production, et l'on évalue la quantité de molécule(s) produite(s) par dosage immunologique sur phase solide in vitro, caractérisé en ce que :
a) la culture cellulaire et éventuellement la stimulation sont effectuées, éventuellement en présence du traceur, dans un récipient qui incorpore la phase solide du dosage sur laquelle sont fixés les éléments de capture spécifiques de la (ou des) molécule(s) à doser, et en ce que
b) lorsque le temps de culture est écoulé, après lavage de la phase solide et incubations des éventuels différents autres réactifs nécessaires au dosage, notamment un substrat du traceur, en présence de la phase solide, on lit le signal émis par le traceur fixé à la phase solide ou par son dit substrat.

2. Procédé selon la revendication 1, caractérisé en ce que dans l'étape b), le traceur et la phase solide sont incubés dans le récipient ayant servi à la culture.

3. Procédé selon les revendications 1 et 2, caractérisé en ce que le signal émis est lu dans le récipient ayant servi à la culture.

4. Procédé selon l'une des revendications 1 à 3, caractérisé en ce que la phase solide consiste en la surface intérieure du récipient de culture sur laquelle sont fixés les éléments de capture spécifiques de la (ou des) molécule(s) à doser.

5. Procédé selon l'une des revendications 1 à 3, caractérisé en ce que la phase solide est constituée par un ou des éléments physiquement indépendants du récipient de culture cellulaire.

6. Procédé selon la revendication 5, caractérisé en ce que la phase solide est constituée par des billes, un élément solidaire d'une pièce de préhension, revêtues desdits éléments de capture spécifiques de la (ou des) molécule(s) à doser.

7. Procédé selon l'une des revendications 1 à 6, dans lequel on dose plusieurs dites molécules caractérisé en ce que :
a) On effectue simultanément la culture cellulaire dans autant de groupes de récipients que de molécules à doser,
b) Sur la phase solide incorporée dans chaque récipient sont fixées plusieurs familles d'anticorps, chacune reconnaissant spécifiquement une des dites molécules et
c) On dose chaque molécule après incubation dans chaque récipient avec un traceur différent spécifique de chaque molécule correspondante.

8. Procédé selon l'une des revendications 1 à 6 dans lequel on dose plusieurs dites molécules caractérisé en ce que :
a) On effectue simultanément la culture cellulaire dans autant de groupes de récipients que de molécules,
b) Sur la phase solide incorporée dans chaque groupe de récipient sont fixés des anticorps de spécificités prédéterminées reconnaissant chacun dans chaque groupe de récipient une molécule différente et
c) On dose chaque molécule après incubation de chaque récipient avec un traceur spécifique de la molécule correspondante, ou un traceur commun qui est un mélange de traceurs spécifiques des différentes molécules.

9. Procédé selon l'une des revendications 1 à 8 dans lequel le dosage immunologique est de type compétition sur phase solide, ou de type sandwich.

10. Procédé selon l'une des revendications 1 à 9 dans lequel l'élément de capture est constitué par des anticorps monoclonaux ou polyclonaux.

11. Procédé selon l'une des revendications 1 à 10 dans lequel l'élément de capture est un antigène ou tout autre ligand, y compris un ligand de récepteur soluble, spécifique de la molécule à doser.

12. Procédé selon l'une des revendications 1 à 11 dans lequel le dosage est révélé par une méthode enzymatique, isotopique, par luminescence, par fluorescence, ou par diffraction néphélométrique.

13. Procédé selon l'une des revendications 1 à 12 dans lequel le composant traceur du dosage est présent dès l'initiation de la culture sur le support ou ajouté ultérieurement.

14. Procédé selon l'une des revendications 1 à 13 dans lequel les médiateurs mesurés sont des cytokines, des hormones, des neuropeptides, des récepteurs solubles, des marqueurs de dégranulation, des peptides extracellulaires ou tout autre médiateur d'effet biologique produit par des cellules in vitro.

15. Procédé selon les revendications 1 à 13, caractérisé en ce que les éléments de particules infectieuses sont constitués par des virus, ou autre germe intracellulaire, ou l'un de leurs éléments antigéniques.

16. Procédé selon l'une des revendications 1 à 15 dans lequel la production de médiateur est spontanée ou stimulée par des agents activateurs spécifiques ou non tels que lectines, lipopolysaccharides, anticorps, antigènes, allergènes, agents pharmacologiques et ligands naturels ou synthétiques de récepteurs cellulaires.

17. Procédé selon l'une des revendications 1 à 16 caractérisé en ce que les anticorps participant au dosage à titre de traceur ou d'élément de capture de la molécule à doser fixés à la phase solide, reconnaissent la molécule à doser par des épitopes n'affectant pas la production de ladite molécule et/ou son activité biologique.

18. Procédé selon les revendications 1 à 17, caractérisé en ce que, lors de la culture, on ajoute au milieu des agents régulateurs de la production de la molécule à doser.

19. Procédé selon l'une des revendications 1 à 18 caractérisé par une désensibilisation du dosage obtenue par un mélange de l'anticorps traceur spécifique de la molécule à doser avec un anticorps non marqué de même spécificité, désensibilisation qui permet grâce à un facteur correctif prédéterminé, d'étendre la gamme de dosage vers les valeurs élevees.

20. Procédé selon l'une des revendications 1 à 19 caractérisé en ce que les échantillons cellulaires à doser sont cultivés simultanément dans un premier milieu de culture comprenant un anticorps traceur spécifique de la molécule à doser et dans un deuxième milieu de culture comprenant un anticorps traceur spécifique de la molécule à doser et un anticorps non marqué de même spécificité.

21. Procédé selon l'une des revendications 1 à 20 Gans lequel les cellules sont représentées par du sang complet, éventuellement dilué. des populations cellulaires purifiées, des cellules extraites de tissus. ou des lignées cellulaires.

22. Procédé selon l'une des revendications 1 à 21 caractérisé en ce que la solution de lavage de la phase solide avant lecture du signal est une solution d'enzyme protéolytique notamment de trypsine.

23. Kit utile dans le procédé selon l'une des revendications 1 à 22, caractérisé en ce qu'il comporte ledit récipient-support de culture cellulaire servant aussi de récipient d'immuno-captation, ladite phase solide sur laquelle sont fixés les éléments de capture de l'immunodosage étant incorporée ou incorporable audit récipient.

24. Kit selon la revendication 23, caractérisé en ce que la phase solide est constituée par la surface intérieure du récipient-support de culture.

25. Kit selon la revendication 24 dans lequel le récipient-support solide est constitué par des puits, éventuellement amovibles, d'une plaque de titration, par des tubes, ou par tout autre récipient de culture cellulaire approprié au dosage immunologique.

26. Kit selon la revendication 23, dans lequel le récipient de culture cellulaire et d'immuno-captation est physiquement indépendant de la phase solide du dosage.

27. Kit selon l'une des revendications 23 à 26, dans lequel sont fournis tous les éléments du dosage, y compris des échantillons standards, le traceur, son éventuel substrat ainsi que des milieux de culture, de dilution cellulaire, de lavage et des agents stimulateurs ou régulateurs de production des paramètres à mesurer.

28. Kit selon l'une des revendications 23 à 27, caractérisé en ce qu'il comprend un traceur et/ou un agent activateur présent(s) sous forme séchée dans les puits de la microplaque.

29. Kit selon l'une des revendications 23 à 28, caractérisé en ce qu'il comprend des échantillons standards de référence présents sous forme séchée dans lesdits récipients, notamment dans les puits de la microplaque.

## Patentansprüche

1. Verfahren zur Bestimmung der Bildung von einem oder mehreren Molekülen wie löslichen Mediatoren oder Elementen von infektiösen Teilchen durch lebende Zellen, bei dem man eine Zellkultivierung und gegebenenfalls eine Stimulierung durchführt, um die genannte Bildung zu induzieren, und die Menge des (der) gebildeten Moleküle(s) ermittelt (berechnet) durch immunologische Bestimmung (Analyse) in fester Phase in vitro, dadurch gekennzeichnet, daß man
a) die Zellkultivierung und gegebenenfalls die Stimulierung gegebenenfalls in Gegenwart eines Tracers in einem Behälter durchführt, der die feste Phase zur immunologischen Bestimmung (Analyse) enthält, an der die für das oder die zu bestimmende(n) Molekül(e) spezifischen Einfangelemente fixiert sind, und
b) nach Ablauf der Kultivierungszeit und nach dem Waschen der festen Phase und der Inkubation der gegebenenfalls für die Bestimmung erforderlichen verschiedenen anderen Reagentien, insbesondere eines Substrats des Tracers, in Gegenwart der festen Phase das Signal abliest, das von dem an der festen Phase fixierten Tracer oder von seinem Substrat emittiert wird.

2. Verfahren nach Anspruch 1, dadurch gekennzeichnet, daß in der Stufe (b) der Tracer und die feste Phase in dem für die Kultivierung verwendeten Behälter inkubiert werden.

3. Verfahren nach den Ansprüchen 1 und 2, dadurch gekennzeichnet, daß das emittierte Signal in dem Behälter abgelesen wird, der für die Kultivierung verwendet worden ist.

4. Verfahren nach einem der Ansprüche 1 bis 3, dadurch gekennzeichnet, daß die feste Phase besteht aus der inneren Oberfläche des Kultivierungsbehälters, an der die für das (oder die) zu bestimmende(n) Molekül(e) spezifischen Einfangelemente fixiert sind.

5. Verfahren nach einem der Ansprüche 1 bis 3, dadurch gekennzeichnet, daß die feste Phase aus einem oder mehreren physikalisch (physisch) voneinander unabhängigen Elementen des Zellkultivierungs-Behälters besteht.

6. Verfahren nach Anspruch 5, dadurch gekennzeichnet, daß die feste Phase aus Kugeln oder einem mit einem Greifelement verbundenen Element besteht, die von dem für die zu bestimmende(n) Molekül(e) spezifischen Einfangelementen bedeckt (beschichtet) sind.

7. Verfahren nach einem der Ansprüche 1 bis 6, bei dem man mehrere der genannten Moleküle bestimmt, dadurch gekennzeichnet, daß man
a) die Zellkultivierung in ebenso vielen Behältergruppen wie zu bestimmenden Molekülen gleichzeitig durchführt,
b) an der in jedem Behälter enthaltenen festen Phase mehrere AntikörperFamilien fixiert, von denen jede eines der genannten Moleküle spezifisch erkennt, und
c) jedes Molekül nach der Inkubation in jedem Behälter mit einem davon verschiedenen, für das entsprechende jeweilige Molekül spezifischen Tracer bestimmt.

8. Verfahren nach einem der Ansprüche 1 bis 6, bei dem man mehrere der genannten Moleküle bestimmt, dadurch gekennzeichnet, daß man
a) die Zellkultivierung in ebenso vielen Gruppen von Behältern wie Molekülen gleichzeitig durchführt,
b) an der in jeder Behältergruppe enthaltenen festen Phase Antikörper mit einer vorgegebenen Spezifität fixiert, die jeweils in einer Behältergruppe ein davon verschiedenes Molekül erkennen, und
c) jedes Molekül nach der Inkubation in jedem Behälter mit einem für das entsprechende Molekül spezifischen Tracer oder mit einem gemeinsamen Tracer, der ein Gemisch der für unterschiedliche Moleküle spezifischen Tracer ist, bestimmt.

9. Verfahren nach einem der Ansprüche 1 bis 8, in dem die immunologische Bestimmung eine solche vom Konkurrenz-Typ an einer festen Phase oder eine solche vom Sandwich-Typ ist.

10. Verfahren nach einem der Ansprüche 1 bis 9, bei dem das Einfangelement aus monoklonalen oder polyklonalen Antikörpern besteht.

11. Verfahren nach einem der Ansprüche 1 bis 10, bei dem das Einfangelement ein Antigen oder irgendein anderer Ligand einschließlich eines für das zu bestimmende Molekül spezifischen löslichen Rezeptorliganden ist.

12. Verfahren nach einem der Ansprüche 1 bis 11, bei dem die Bestimmung (Analyse) mittels einer Enzym-, Isotopen-, Lumineszenz-, Fluoreszenz- oder nephelometrische Beugungsmethode erfolgt.

13. Verfahren nach einem der Ansprüche 1 bis 12, bei dem die Tracer-Komponente der Bestimmung ab Initiierung der Kultivierung auf dem Träger vorliegt oder später zugegeben wird.

14. Verfahren nach einem der Ansprüche 1 bis 13, bei dem die bestimmten Mediatoren Cytokine, Hormone, Neuropeptide, lösliche Rezeptoren, Degranulationsmarker, extrazelluläre Peptide oder irgendein anderer Mediator mit biologischer Wirkung, der von Zellen in vitro gebildet wird, sind.

15. Verfahren nach den Ansprüchen 1 bis 13, dadurch gekennzeichnet, daß die Elemente von infektiösen Teilchen aus Viren oder einem anderen intrazellulären Keim oder einem ihrer antigenen Elemente bestehen.

16. Verfahren nach einem der Ansprüche 1 bis 15, bei dem die Bildung des Mediators spontan erfolgt oder stimuliert wird durch spezifische oder nichtspezifische Aktivatoren, wie Lectine, Lipopolysaccharide, Antikörper, Antigene, Allergene, pharmakologische Agentien und natürliche oder synthetische Liganden von Zellrezeptoren.

17. Verfahren nach einem der Ansprüche 1 bis 16, dadurch gekennzeichnet, daß die Antikörper, die an der Bestimmung (Analyse) als Tracer oder als Element zum Einfangen des zu bestimmenden Moleküls beteiligt sind und an der festen Phase fixiert sind, das zu bestimmende Molekül durch Epitope erkennen, welche die Bildung des genannten Moleküls und/oder seine biologische Aktivität nicht beeinflussen.

18. Verfahren nach einem der Ansprüche 1 bis 17, dadurch gekennzeichnet, daß man bei der Kultivierung dem Medium Agentien zur Regulierung der Bildung des zu bestimmenden Moleküls zugibt.

19. Verfahren nach einem der Ansprüche 1 bis 18, dadurch gekennzeichnet, daß die erhaltene Bestimmung desensibilisiert wird durch ein Gemisch von Tracer-Antikörpern, das für das zu bestimmende Molekül spezifisch ist, mit einem nicht-markierten Antikörper der gleichen Spezifität, wobei die Desensibilisierung dank eines vorgegebenen Korrekturfaktors die Erweiterung des Meßbereiches in Richtung auf höhere Werte erlaubt.

20. Verfahren nach einem der Ansprüche 1 bis 19, dadurch gekennzeichnet, daß die zu bestimmenden Zellproben gleichzeitig in einem ersten Kulturmedium, das einen für das zu bestimmende Molekül spezifischen Tracer-Antikörper enthält, und in einem zweiten Kulturmedium, das einen für das zu bestimmende Molekül spezifischen Tracer-Antikörper und einen nicht-markierten Antikörper mit der gleichen Spezifität enthält, kultiviert werden.

21. Verfahren nach einem der Ansprüche 1 bis 20, bei dem die Zellen repräsentiert werden durch gegebenenfalls verdünntes Vollblut, gereinigte Zellpopulationen, aus Gewebe extrahierte Zellen oder Zellinien.

22. Verfahren nach einem der Ansprüche 1 bis 21, dadurch gekennzeichnet, daß die Lösung zum Waschen der festen Phase vor dem Lesen des Signals eine Lösung eines proteolytischen Enzyms, insbesondere von Trypsin, ist.

23. Kit, wie er in dem Verfahren nach einem der Ansprüche 1 bis 22 verwendbar ist, dadurch gekennzeichnet, daß er umfaßt den genannten Behälter-Träger für die Zellkultivierung, der auch als Immunoeinfang-Behälter dient, und die genannte feste Phase, an der die Einfangelemente zur Immunbestimmung fixiert sind, die dem Behälter einverleibt worden ist oder diesem einverleibt werden kann.

24. Kit nach Anspruch 23, dadurch gekennzeichnet, daß die feste Phase aus der inneren Oberfläche des Behälter-Trägers für die Kultivierung besteht.

25. Kit nach Anspruch 24, in dem der feste Behälter-Träger aus gegebenenfalls auswechselbaren Vertiefungen einer Titrationsplatte, Reagensgläsern oder irgendeinem anderen Behälter für die Zellkultivierung, der für die immunologische Bestimmung geeignet ist, besteht.

26. Kit nach Anspruch 23, in dem der Behälter für die Zellkultivierung und für das Immuneinfangen von der festen Phase für die Bestimmung (Analyse) physikalisch (physisch) unabhängig ist.

27. Kit nach einem der Ansprüche 23 bis 26, in dem alle Elemente für die Bestimmung (Analyse) einschließlich der Standard-Proben, der Tracer, sein eventuelles Substrat sowie die Medien zur Kultivierung, zur Zellverdünnung, zum Waschen und Stimulator- oder Regulatoragentien für die Bildung der zu bestimmenden Parameter vereinigt sind.

28. Kit nach einem der Ansprüche 23 bis 27, dadurch gekennzeichnet, daß er einen Tracer und/oder einen Aktivator umfaßt, der (die) in getrockneter Form in den Vertiefungen der Mikrotitrationsplatte vorhanden ist (sind).

29. Kit nach einem der Ansprüche 23 bis 28, dadurch gekennzeichnet, daß er Referenz-Standardproben umfaßt, die in getrockneter Form in den genannten Behältern, insbesondere in den Vertiefungen der Mikrotitrationsplatte, vorhanden sind.

## Claims

1. Method for evaluating the production by living cells of molecule(s) such as soluble mediators or components of infectious particles, in which method cell culturing is performed, and optionally a stimulation, to induce the said production, and the quantity of molecule(s) produced is evaluated by solid-phase immunoassay in vitro, characterized in that:
a) the cell culturing and, where appropriate, the stimulation are performed, optionally in the presence of the tracer, in a vessel which incorporates the solid phase of the assay to which are bound the capture components specific for the molecule(s) to be assayed, and characterized in that
b) when the culture period has elapsed, after washing of the solid phase and incubation of the various other possible reagents needed for the assay, in particular a substrate for the tracer in the presence of the solid phase, the signal emitted by the tracer bound to the solid phase or by its said substrate is measured.

2. Method according to Claim 1, characterized in that, in step b), the tracer and the solid phase are incubated in the vessel used for culturing.

3. Method according to Claims 1 and 2, characterized in that the signal emitted is measured in the vessel used for culturing.

4. Method according to one of Claims 1 to 3, characterized in that the solid phase consists of the inner surface of the culture vessel, to which are bound the captured components specific for the molecule(s) to be assayed.

5. Method according to one of Claims 1 to 3, characterized in that the solid phase consists of one or more components physically independent of the cell culture vessel.

6. Method according to Claim 5, characterized in that the solid phase consists of beads, or a component firmly attached to a part which can be grasped, coated with the said capture components specific for the molecule(s) to be assayed.

7. Method according to one of Claims 1 to 6, in which several said molecules are assayed, characterized in that:
a) Cell culturing is performed simultaneously in as many vessels as there are molecules to be assayed,
b) To the solid phase incorporated in each vessel, there are bound several families of antibodies, each one specifically recognizing one of the said molecules, and
c) Each molecule is assayed after incubation in each vessel with a different tracer specific for each corresponding molecule.

8. Method according to one of Claims 1 to 6, in which several said molecules are assayed, characterized in that:
a) Cell culturing is performed simultaneously in as many groups of vessels as there are molecules,
b) To the solid phase incorporated in each group of vessels there are bound antibodies of predetermined specificities each recognizing in each group of vessels a different molecule, and
c) Each molecule is assayed after incubation of each vessel with a tracer specific for the corresponding molecule, or a common tracer which is a mixture of tracers specific for the different molecules.

9. Method according to one of Claims 1 to 8, in which the immunoassay is of the solid-phase competitive type or of the sandwich type.

10. Method according to one of Claims 1 to 9, in which the capture component consists of monoclonal or polyclonal antibodies.

11. Method according to one of Claims 1 to 10, in which the capture component is an antigen or any other ligand, including a ligand of a soluble receptor, specific for the molecule to be assayed.

12. Method according to one of Claims 1 to 11, in which the assay is visualized by an enzymatic or isotopic method, by luminescence, by fluorescence or by nephelometric diffraction.

13. Method according to one of Claims 1 to 12, in which the tracer component of the assay is present from the start of culturing on the support or added subsequently.

14. Method according to one of Claims 1 to 13, in which the mediators measured are cytokines, hormones, neuropeptides, soluble receptors, degranulation markers, extracellular peptides or any other mediator of a biological effect produced by cells in vitro.

15. Method according to one of Claims 1 to 13, characterized in that the components of infectious particles consist of viruses, or some other intracellular microorganism, or one of their antigenic components.

16. Method according to one of Claims 1 to 15, in which the production of mediator is spontaneous, or stimulated with activating agents, specific or otherwise, such as lectins, lipopolysaccharides, antibodies, antigens, allergens, pharmacological agents and natural or synthetic ligands of cell receptors.

17. Method according to one of Claims 1 to 16, characterized in that the antibodies participating in the assay as a tracer or capture component for the molecule to be assayed, and which are bound to the solid phase, recognize the molecule to be assayed by epitopes which do not affect the production of the said molecule and/or its biological activity.

18. Method according to Claims 1 to 17, characterized in that, during culturing, agents that regulate the production of the molecule to be assayed are added to the medium.

19. Method according to one of Claims 1 to 18, characterized by a desensitization of the assay, obtained by a mixture of the tracer antibody specific for the molecule to be assayed with an unlabelled antibody of the same specificity, which desensitization permits the range of assay to be extended to high values by means of a predetermined correction factor.

20. Method according to one of Claims 1 to 19, characterized in that the cell samples to be assayed are cultured simultaneously in a first culture medium comprising a tracer antibody specific for the molecule to be assayed and in a second culture medium comprising a tracer antibody specific for the molecule to be assayed and an unlabelled antibody of the same specificity.

21. Method according to one of Claims 1 to 20, in which the cells are represented by whole blood, optionally diluted, purified cell populations, cells extracted from tissues or cell lines.

22. Method according to one of Claims 1 to 21, characterized in that the solution for washing the solid phase before measuring the signal is a solution of a proteolytic enzyme, in particular of trypsin.

23. Kit which is appropriate to the method according to one of Claims 1 to 22, characterized in that it contains the said support vessel for cell culture serving also as a vessel for immunocapture, the said solid phase to which the capture components of the immunoassay are bound being incorporated or being capable of incorporation in the said vessel.

24. Kit according to Claim 23, characterized in that the solid phase consists of the inner surface of the culture support vessel.

25. Kit according to Claim 24, in which the solid support vessel consists of wells, optionally removable, of a titration plate, or consists of tubes or any other cell culture vessel suitable for immunoassay.

26. Kit according to Claim 23, in which the vessel for cell culture and immunocapture is physically independent of the solid phase of the assay.

27. Kit according to one of Claims 23 to 26, in which all the components of the assay, including standard samples, the tracer, its substrate, where appropriate, as well as culture media, cell dilution media, washing media and agents that stimulate or regulate production of the parameters to be measured, are supplied.

28. Kit according to one of Claims 23 to 27, characterized in that it comprises a tracer and/or an activating agent present in dried form in the wells of the microplate.

29. Kit according to one of Claims 23 to 28, characterized in that it comprises standard reference samples present in dried form in the said vessels, in particular in the wells of the microplate.
